# EUROPEAN PATENT SPECIFICATION

(11) **EP 1 919 390 B1**
(45) Date of publication and mention of the grant of the patent: **19.12.2012**
(21) Application number: 06765211.5
(22) Date of filing: 04.08.2006
(51) Int. Cl.: A61B 19/00, G06F 3/14

(54) **COMPUTER ASSISTED SURGERY SYSTEM**
COMPUTERGESTÜTZTES CHIRURGISCHES SYSTEM
SYSTEME CHIRURGICAL ASSISTE PAR ORDINATEUR

(30) Priority: 05.08.2005 EP 05254925
(43) Date of publication of application: 14.05.2008
(73) Proprietor: DePuy Orthopädie GmbH, 85551 Heimstetten (DE)
(72) Inventor: HAECHLER, Joerg, 6340 Baar (CH); BURGER, Thorsten, 80804 München (DE); WARNOCK, Alex, Aberdeenshire AB12 5XL (GB); CRONIN, Dave, San Francisco, CA 94105 (US); LEMOINE, Doug, San Francisco, CA 94105 (US); GUYOT, Noah, San Francisco, CA 94105 (US); BALAITY, Andrew, Warsaw, IN 46581 (US); HAYES, Johanna, Warsaw, IN 46581 (US)
(74) Representative: Alton, Andrew
(86) International application number: PCT/GB2006/002907
(87) International publication number: WO 2007/017642

(56) References cited:
- EP-A- 1 406 203
- WO-A-99/59106
- WO-A-03/045222
- WO-A-2004/001569
- WO-A-2004/016182
- US-A1- 2003 208 122
- US-A1- 2003 220 557
- US-A1- 2004 169 673

## Description

The present invention relates to computer assisted, or aided, surgery ("CAS"), and in particular to a user interface for a computer assisted surgery system.

A graphical user interface (GUI) provide a mechanism by which a user can interact with a computer system. A GUI can display information to users and also present a number of control elements or widgets which the user can select or operate so as to interact with the user interface and control the computer system. A large amount of information can be displayed to the user in a wide variety of formats and with a large number of widgets or other entities. Hence, it can be difficult for a user to identify and focus on the information requiring immediate attention, or of most relevance, and also to determine the appropriate widget with which to interact with and control the computer system in the most efficient manner.

A GUI can be used to interact with a computer assisted, or aided, surgery (CAS) system. Such systems can be used to assist a surgeon in a number of ways while carrying out a surgical procedure, such as, by way of example, displaying images to guide performance of steps of the surgical procedure (image guided surgery or IGS). CAS systems can provide improved accuracy in performing a surgical procedure and can also speed up the surgical procedure. Hence, it is important that a GUI for a CAS system provide information and control in a format that is easy for the surgeon to use.

A CAS system is there to assist the surgeon and not to replace the surgeon's decision making and experience. Therefore, it is also important that a GUI not overly constrain the surgeon's control of the surgical procedure.

International patent application publication no WO 2004/016182 A1 describes a graphical user interface and a system for performing an orthopaedic implant procedure which uses an expert system driven control application to display a series of screens organized in a sequential series providing information relating to steps required to perform a procedure. However, various different types of entities are presented in different regions of the GUI making it difficult for the user to easily and clearly identify and prioritise the parts of the GUI requiring attention. The expert system driven control also reduces the flexibility of the system and constrains the surgeon's behaviour.

US patent application publication no. 2004/0169673 describes an extension of the above described graphical user interface. A series of pages associated with steps to be carried out are presented and a positive or negative indication for each step can be input via an icon displayed in a same region of the display pages. This allows a foot-operated input device to be used to control the CAS system. Hence, although this provides a consistently presented icon in the user interface, the yes/no answering does not allow the surgeon extensive freedom in how they use the CAS system to carry out the procedure and so flexibility is sacrificed for ease of navigation through the screens of the user interface.

WO 03/045222 describes a user interface for a CAS system having an image display area which can display a plurality of 2D and 3D images, an information display portion to the side of the image display area and tool bars above the image display portion. Information about a currently displayed image is displayed to the side of the image display area and tool buttons of the user interface are located within the image display area.

WO 2004/001569 describes a user interface for a CAS system which can display a pair of images extending across a top part of the UI with an information area below them and some control buttons below the information area. Information is displayed within the image areas and a control element is present in the bottom right hand corner of the image areas.

US 2003/208122 describes a user interface for a CAS system which can display a pair of images extending across a top part of the UI, each image area including control buttons, with information about the current surgical step displayed below the right hand image and a vertical column of control buttons to the right of that information.

Hence, there is a need for a GUI for a CAS system which is easy for the surgeon to use so that the benefits of a CAS system are not lost by the complexity of interacting with and operating the CAS system. It is also important to provide flexibility in the surgical procedure so that the CAS system is driven by the surgeon and not *vice versa..*

According to a first aspect of the present invention, there is provided a graphical user interface of a computer assisted surgery system for use by a surgeon during steps of a workflow for a surgical procedure being carried out on a patient, the user interface comprising: a working portion extending across the user interface in which a plurality of images of different views of a body part of the patient are displayed during the steps of the workflow, and in which the plurality of images are separate from other elements of the user interface; an information portion positioned below the working portion and extending across the user interface in which information relating to a current step of the workflow illustrated by the images currently displayed in the working portion is displayed; a control element which can be activated to import a surgeon profile of the surgeon, wherein the surgeon profile includes data relating to how the surgical workflow is customised, so as to allow the computer assisted surgery system to be customised according to the preferences of the surgeon; and a control bar positioned below the information portion and extending across the user interface and including a plurality of control elements which can be operated by a user of the computer assisted surgery system to operate the computer assisted surgery system.

By utilising a layered approach in which certain types of functions of the user interface are consistently presented in the same layer, it is easier for the surgeon to use the user interface as the surgeon can more reliably and efficiently identify the immediately required elements of the user interface. Also as a consistent layout is used for all the screens associated with carrying out surgical procedures, there is continuity of comprehension between different stages of the surgical procedures, such as planning, registration and navigation. The consistent presentation and layout also means that there is no need to constrain how the surgeon uses the user interface and so the surgeon still retains freedom to conduct the surgical procedure as they see fit.

The system can be a computer assisted orthopaedic surgery system. Such a system is specially configured to carry out orthopaedic surgical procedures, such as joint replacement or arthroplasty, spinal procedures. In particular the system can be configured to carry out hip, knee and/or shoulder replacement procedures using prosthetic implants.

The user interface can include a header. The header can include an indication of the surgeon currently using the system and/or of the current patient.

The working portion and/or information portion and/or control bar and/or headed can each extend across the whole of the width of the user interface. The working portion can have a constant height. The information portion can have a constant height. The height of the working portion can be greater than the height of the information portion. The height of the information portion can be greater than the height of the control bar and/or header.

Control elements which cause the graphical user interface to transition between screens can be located in the same region of the control bar. Hence, the user does not need to go looking for the control elements on a new screen in order to navigate between different screens of the user interface. The navigation control elements can include a forward button and a back button.

The control bar can include a control element for activating a tool bar. The tool bar can provide a plurality of control elements which can be used to control tools provided by the graphical user interface.

The working portion can have at least two different image display modes. In a first display mode the images of different views of the body part can be different sizes. A first image can be a main image and other images can be minor images which are smaller than the main image. The minor images can be displayed as thumb nails. In a second display mode the images of different views of the body part can be substantially the same size. The images can take up substantially the same amount of the screen, either in area or height or width, and each image can have a different level of magnification of the body part. The control bar can include a control element for switching between the first and second display modes. A single toggle control element can be provided for switching between images. Separate control elements can be provided to allow either display mode to be selected. The first and second display modes can each display at least two different views and can also display at least three, or more, different views. The number of different views in the first and second display modes can be different.

In the first display mode, the user can change the main image by selecting a one of the smaller images. The smaller image can be selected by touching the smaller image on the screen. The main image is then made the main image and the main image is made a smaller image. Hence, the surgeon can easily display the view of the body part most relevant to their current needs by swapping between the view displayed in the main and the view or views displayed in the smaller thumbnail images.

The control bar can include a control element which can be operated to overlay a list of actions. The list of actions can indicate which actions have been carried out and which actions are to be carried out. The list of actions can define a sequence of actions to be carried out according to a surgical workflow. The actions can include registration actions, planning actions, surgical actions and/or navigated surgical actions. The list of actions can include actions that can be added to a workflow, but are not currently part of the workflow. Actions that can be performed can be added to the actions to be performed in the workflow by selecting the action or actions from the list. Actions that can be added to the workflow can be displayed in a different manner to actions that have been performed and/or that are intended to be performed in a current workflow. For example, actions that are not currently part of the workflow can be grayed out or otherwise distinguished from actions that are part of the current work flow.

An item can be selected from the list of actions to navigate the graphical user interface to a screen associated with the selected item. Hence, the surgeon can jump directly to a particular step in the workflow or to a particular screen of the user interface without having to navigate through intervening steps or screens. The list can be an intelligent list which prevents navigation to screens relating to a part of the workflow which cannot be used yet because a preceding part of the workflow has not yet been completed. For example, the list can prevent navigating to screens relating to surgical navigation unless registration has been carried out.

The control bar can include a control element which can be activated to allow an annotation associated with a current step to be added. This allows the surgeon to annotate or otherwise add comments to stages or steps of the workflow. The annotations or comments can be used subsequently in reporting activities or can provide guidance for other users. The annotation or comments can be input verbally and converted into text via a speech to text converter or speech recognition process. An audio file of the annotation or comments can be stored and/or a text file.

The graphical user interface can include a control element which can be activated to cause the user interface to output guidance relating to a current step. This provides assistance to the surgeon which explains or clarifies a current step of the workflow, or otherwise provides information relating to the procedure being carried out. The guidance can be context sensitive so that the information provided is related to the current stage or step of the procedure. The guidance can be provided in various different forms, including text, images, sequences of images, animations, models, video, 3d animations, audio and any combination thereof. The guidance can be derived from an expert system which provides advice to the surgeon based on the context of the current step of the procedure and/or on surgeon input such as questions or other requests for guidance.

The graphical user interface can include a user selection screen via which a current user of the computer assisted surgery system can be identified. This allows the surgeon currently using the system to be associated with the surgical procedure being carried out.

The surgeon profile can include a number of data items specifying data relating to the surgeon and/or data relating to how the CAS system is configured and/or data relating to how the user interface is configured. The user interface can automatically import a surgeon profile on detecting that a media item bearing a surgeon profile is accessible to the user interface.

The graphical user interface can include a control element which can be activated to select a standard workflow for carrying out the surgical procedure. Hence, the surgeon can select to carry out a surgical procedure using a standard workflow which will guide the surgeon through a predefined sequence of steps in order to carry out the surgical procedure in a standard manner.

The graphical user interface can include a control element which can be activated to select a customised workflow for carrying out the surgical procedure. A customised workflow or workflows can be stored and selected via the user interface so that the user interface can guide the surgeon through a non-standard sequence of steps or using non-standard parameters or setting in order to carry out the surgical procedure in a customised manner preferred by the surgeon or appropriate for the current patient.

The graphical user interface can include a screen including a plurality of control elements operable to set a plurality of settings used by a workflow for carrying out the surgical procedure. The plurality of settings can be used to customise or configure a standard workflow according to the surgeon's preferred practice. The settings can be used to configure the properties associated with individual steps of the user interface.

The graphical user interface can include a screen including a plurality of control elements operable to define a strategy to be used during a workflow for carrying out the surgical procedure. In this way the surgeon can customise or tailor the workflow to be carried out In a different way, for example, by selecting to omit or include certain steps or stages of the procedure, such as an implant optimization stage, or to change the sequence of steps or stages of the procedure.

The graphical user interface can include a screen including a plurality of control elements operable to determine the display mode of the working portion of the graphical user interface at different steps in the workflow for carrying out the surgical procedure. Hence, the surgeon can select what display modes to be used at different steps and also what images are to be displayed in the different display modes and properties of the images to be displayed, such as their magnification or the direction of view of the image of the body part.

The graphical user interface can include a registration screen allowing a user to capture or recapture a registration point on a body part by selecting a graphical indication of the point. In this way, the surgeon can select to capture only a particular point in order to improve registration, rather than having to capture all, or a large number of, points used in registration.

The graphical user interface can include a registration screen which includes a progress meter which provides a linear indication of the proportion of completion of the collection of points on the surface of the body part. The progress meter can be in the form of a bar. The progress meter can display a numerical value indicating the proportion of completion of the collection of registration points. This provides a visual indication to the surgeon of how many more points need collecting for the registration process.

A first image in the working portion can be an image of the surface of a body part. The position of a point on the surface of the body part can be indicated by a target. The target can be in the form of cross hairs. A second image in the working portion can be a cross section through the body part along lines defined by the target. The lines can be a one of, or both, the cross hairs. This allows the surgeon to more easily identify the spatial relationship between the cross sectional image or images and the surface image of the body part.

Parts of the target can be displayed in different colours and the cross section or sections can be colour coded with the respective parts of the target, for example cross hairs. This provides further visual guidance as to the spatial relationship between the surface image and the displayed cross sections.

At least one screen can include a widget for setting a value of a planning property. The widget can include a first control element for incrementing the value and/or a second control element for decrementing the value and/or a display of the numerical amount of the current value. A widget can be provided for every degree of freedom required by the planning. Widgets can be provided for selecting the planned position and/or the orientation. Widgets can be provided to select the size of the implant. Multiple widgets can be provided for selecting different positional properties and/or multiple widgets can be provided for selecting different angular properties. The or each widget can further comprise text describing the planning property.

The or each widget can be displayed in the working portion of the user interface. The or each widget can be displayed separately from the images of the body parts in the display portion. When there are a plurality of widgets, the plurality of widgets can be displayed in a group which is separate to the images of the body parts. The plurality of widgets can be grouped in a row or in a column or in an array. This helps to segregate control elements from the body part images so that the widgets do not interfere with visualization of the body parts and are consistently located such that the surgeon can easily find the appropriate widget in the user interface.

In the first display mode, a plurality of control elements can be displayed to one side of the displayed images. The plurality of control elements can be arranged in a column or an array. The plurality of control elements can be displayed on the left had side or the right hand side of the displayed images. In the second display mode, a plurality of control elements can be displayed above and/or below the displayed images. The plurality of control elements can be arranged in a row or an array.

The control elements can be spatially associated with the image which most clearly displays the property that the control element relates to. The or each control element related to a property displayed in a first image can be positioned adjacent the first image and the or each control element related to a different property displayed in a second image can be positioned adjacent the second image. And similarly depending on the number of images, control elements and properties. In this way it is easier for the surgeon to locate the control element used to adjust the property displayed the images.

At least one control element can be displayed in the information portion. More than one control element can be displayed in the information portion. The or each control element can be displayed in a region of the information portion which is not adjacent to the images displayed in the working portion. Preferably, only information relating to the displayed images is displayed in the region of the information portion adjacent the region of the working portion in which images are displayed.

The user interface can include at least one navigation screen. The or each navigation screen can include at least one widget providing a visual comparison of the determined position of an entity with the planned position of the entity. The entity may be an implant, part of an implant, a tool, an instrument or any entity used in a surgical procedure. The or each widget can graphically displaying a target or planned value for a planning property and/or a current value for the planning property and/or the numerical value of the target or planned value. In this way, the surgeon can easily navigate the entity and determine how closely the position of the entity corresponds to its panned position. The navigation widgets can be positioned as indicated above for control elements in general.

The or each widget can further includes a graphical indication of a range of acceptable target values. The range of acceptable target values can have been set by a user. The range of acceptable target or planned values can be graphically indicated by a coloured band in the widget. The coloured band can have a different colour to a background part of the widget surrounding the coloured band. The widget can include a needle, pointer, line or other movable indicator whose position changes to indicate the current position of the navigated entity. The indicator can move over a scale and the coloured band can be provided on the scale.

At least some of the or each widget can change colour when the current value of the planning property is within the range of acceptable target values. For example, at least a part of the widget can change from red to green when the current value of the planning property is within the range of acceptable target values.

The graphical user interface can include a planning screen. The planning screen can include a widget for adjusting the planned position of an implant over a plane. The widget can include four control elements in a cruciform arrangement. Each control element can be used to adjust the planned position of the implant along two mutually perpendicular axes of the plane. A first pair of opposed control elements can relate to a first axis and a second pair of opposed control elements can relate to a second axis.

The surgical procedure comprise a plurality of general steps. A general step may relate to a navigation step, a planning step or a navigate surgical step. The surgical procedure may include a plurality of general steps of each type and/or of different types in a variety of orders, depending on the workflow for the surgical procedure. Each general step can comprise a plurality of sub steps. Each of a plurality of screens of the user interface can be associated with a sub step. Each screen associated with a sub step can include an indication of the proportion of completion of the general step. The indication can be presented in numerical format. For example, the numerical format can be the number of the current sub step compared to the total number of sub steps in the general step.

The user interface can include a visual tracking status indicator. The indicator can provide a visual indication whether entities are reliably being tracked by a tracking part of the computer assisted surgery system. The tracking status indicator can be displayed in a header part of the user interface. The tracking status indicator can change colour when at least one entity is not being tracked reliably. The tracking status indicator can change colour from green to red. The tracking status indicator can change colour to a plurality of different colours, depending on the number of entities not being reliably tracked. For example, the indicator can change colour from green to orange if one entity is not being reliably tracked, and then change colour to red, if two entities are not being reliably tracked.

A separate tracking status indicator can be provided for each entity currently being tracked for a current step of the procedure.

The tracking status indicator can also display a visual indication of the identity of the entity which is not being tracked reliably. For example a character can be displayed which identifies the entity not being reliably tracked. The character can be the initial letter of the name of the entity not being reliably tracked. For example, "I" for an instrument, "P" for a pointer, "F" for a femoral marker, "T" for a tibial marker, "P" for a pelvic marker, etc. The tracking status indicator can include a graphical representation of the entity not being reliably tracked. For example, a graphical representation of a pointer, femur, tibia, instrument or pelvis can be displayed.

The graphical user interface can include at least one screen including a control element which can be operated to generate a report on the surgical procedure. Alternatively, a report can be automatically generated using a setting of the user interface. The report can include at least some data entered into the computer assisted surgery system via the user interface or derived from data otherwise provided by the user interface or the surgeon's interaction with the user interface. For example, the data may relate to the workflow used, the implants used, the planned position of the implants, the actual navigated position of the implants, blood loss, anaesthesia information, information for follow up treatment (*e.g.* physiotherapy), comments or annotations entered by the surgeon and any combination thereof. Hence, the user interface allows the surgeon to prepare at least a part of a report on the surgical procedure.

The graphical user interface can further include a control element which can be operated to save the report or export the report. The report can be exported from the computer assisted surgery system to a storage device over a network. The network may be part of a hospital information services network. The storage device may store patient records. Hence, the report or at least part of the report is made available to other medical professionals.

According to a further aspect of the invention, there is provided a computer assisted surgery system for use by a surgeon during steps of a workflow for a surgical procedure being carried out on a patient, the computer assisted surgery system including at least one data processing device, a display device and at least one memory holding computer program instructions which can configure the data processing device to display a graphical user interface on the display device, the graphical user interface comprising: a working portion extending across the user interface in which a plurality of images of different views of a body part of the patient are displayed during the steps of the workflow, and in which the plurality of images are separate from other elements of the user interface; an information portion positioned below the working portion and extending across the user interface in which information relating to a current step of the workflow illustrated by the images currently displayed in the working portion is displayed; a control element which can be activated to import a surgeon profile of the surgeon, wherein the surgeon profile includes data relating to how the surgical workflow is customised, so as to allow the computer assisted surgery system to be customised according to the preferences of the surgeon; and a control bar positioned below the information portion and extending across the user interface and including a plurality of control elements which can be operated by the surgeon to operate the computer assisted surgery system.

The computer assisted surgery system can include computer program instructions which can configure the data processing device to display a graphical user interface having any of the preferred features of the user interface mentioned above.

According to a further aspect of the invention, there is provided a method for providing a graphical user interface of a computer assisted surgery system for use by a surgeon during steps of a workflow for a surgical procedure being carried out on a patient, the method comprising: displaying a plurality of images of different views of a body part of the patient during the steps of the workflow in a working portion of the user interface extending across the user interface, and in which the plurality of images are separate from other elements of the user interface; displaying information relating to a current step of the workflow illustrated by the images currently displayed in the working portion in an information portion of the user interface positioned below the working portion and extending across the user interface; displaying a plurality of control elements in a control bar of the user interface positioned below the information portion and extending across the user interface; importing a surgeon profile of the surgeon, wherein the surgeon profile includes data relating to how the surgical workflow is customised, so as to allow the computer assisted surgery system to be customised according to the preferences of the surgeon; and operating the computer assisted surgery system in response to operation of control elements displayed in the user interface by the surgeon according to the customised workflow.

The method can further include method steps to provide any of the features of the user interface mentioned above.

According to a further aspect of the invention, there is provided a computer program product comprising a computer readable medium bearing computer program code providing instructions which can be carried out by a data processing device to provide a graphical user interface of a computer assisted surgery system for use by a surgeon during steps of a workflow for a surgical procedure being carried out on a patient, the instructions including instructions to: display a plurality of images of different views of a body part of the patient during the steps of the workflow in a working portion of the user interface and extending across the user interface, and in which the plurality of images are separate from other elements of the user interface; display information relating to a current step of the workflow illustrated by the images currently displayed in the working portion in an information portion of the user interface positioned below the working portion and extending across the user interface; display a plurality of control elements in a control bar of the user interface positioned below the information portion and extending across the user interface; import a surgeon profile of the surgeon, wherein the surgeon profile includes data relating to how the surgical workflow is customised, so as to allow the computer assisted surgery system to be customised according to the preferences of the surgeon; and operate the computer assisted surgery system in response to operation of control elements displayed in the user interface by the surgeon according to the customised workflow.

The computer program product can include further instructions to provide a graphical user interface including any of the features of the user interface mentioned above.

An embodiment of the invention will now be described, by way of example only, and with reference to the accompanying drawings, in which:
Figure 1 shows a schematic illustration of a computer assisted surgery system according to the invention;
Figure 2 shows a schematic software architecture of the system shown in Figure 1 and including a user interface according to the invention;
Figure 3 shows a high level flow chart illustrating a method of interacting with the system shown in Figure 1 using the user interface of the invention;
Figure 4 shows a process flow chart illustrating a procedure selection part of the method illustrated in Figure 3;
Figure 5 shows a process flow chart illustrating a workflow customization part of the method illustrated in Figure 4;
Figure 6 shows a process flow chart illustrating a registration part of the method illustrated in Figure 3;
Figure 7 shows a process flow chart illustrating a planning part of the method illustrated in Figure 3;
Figure 8 shows a process flow chart illustrating a navigation part of the method illustrated in Figure 3;
Figure 9 is a screen shot of a screen of the user interface of the invention illustrating the overall layout of the user interface;
Figure 10 is a screen shot of a screen of the user interface illustrating the provision of a tool bar;
Figure 11 is a screen shot of an initial screen of the user interface;
Figure 12 is a screen shot of a workflow selection screen of the user interface;
Figure 13 is a screen shot of a settings screen of the user interface used during workflow customization;
Figure 14 is a screen shot of a strategy screen of the user interface used during a workflow customization;
Figure 15 is a screen shot of a view selection screen of the user interface used during a workflow customization;
Figure 16 is a screen shot of a screen of the user interface used during registration;
Figure 17 is a screen shot of a screen of the user interface used during registration and illustrating an index feature of the user interface;
Figure 18 is a screen shot of a screen of the user interface used during registration;
Figure 19 is a screen shot of a verification screen of the user interface used to verify registration of a body part;
Figure 20 is a screen shot of a planning screen of the user interface illustrating a first display modality;
Figure 21 is a screen shot of the planning screen shown in Figure 20 in a second display modality;
Figure 22 is a screen shot of a further planning screen of the user interface illustrating a first display modality;
Figure 23 is a screen shot of the further planning screen shown in Figure 22 in a second display modality;
Figure 24 is a screen shot of a further planning screen illustrating a guidance feature of the user interface;
Figure 25 is a screen shot of a yet further planning screen of the user interface;
Figure 26 is a screen shot of a navigation screen of the user interface illustrating a first display modality;
Figure 27 is a screen shot of the navigation screen shown in Figure 26 using a second display modality;
Figure 28 is a screen shot of a verification screen of the user interface;
Figure 29 shows a screen shot of a further navigation screen of the user interface;
Figure 30 shows a part of the screen shot shown in Figure 29, illustrating a targeting feature;
Figure 31 shows a process flow chart illustrating a reporting part of the method illustrated in Figure 3; and
Figure 32 is a schematic illustration of the hardware architecture of the data processing apparatus of the system shown in Figure 1.

Similar items in different Figures share common reference numerals unless indicated otherwise.

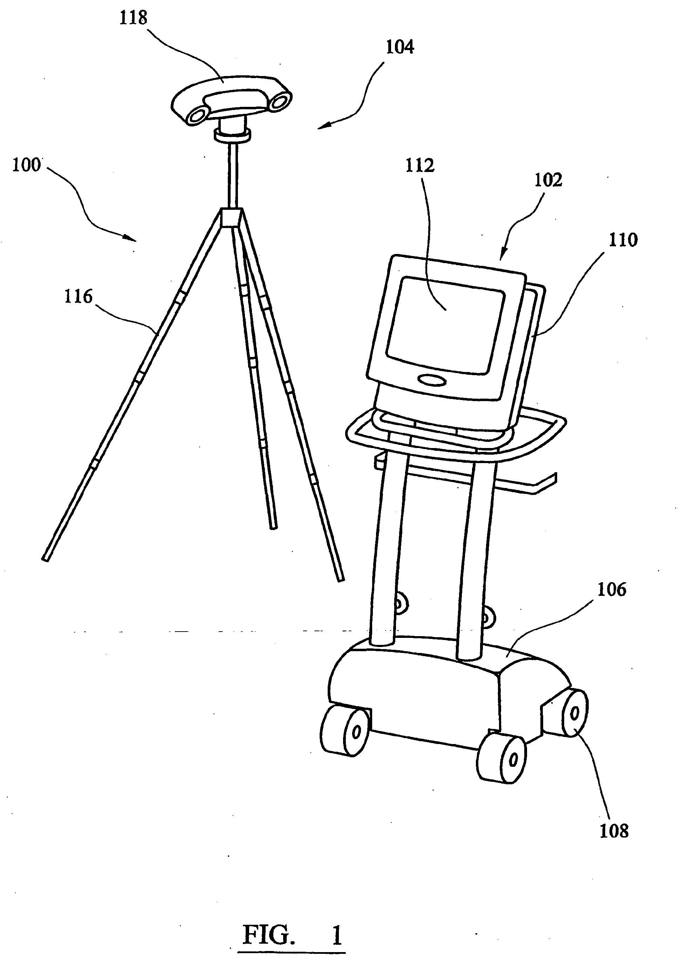

With reference to Figure 1 there is shown a schematic illustration of a computer assisted surgery (CAS) system 100 according to the invention. The CAS system 100 includes a computer based moveable workstation 102 and a camera part 104 of a tracking system. Workstation 102 has a base 106 including trolley wheels 108. The base 106 supports a housing 110 enclosing a computer and having a touch sensitive display device 112. Workstation 102 can also be provided with a foot pedal including three or more switches via which a user can control the workstation 102. Workstation 102 can also be provided with other input devices, such as a mouse or a joystick as required. Other modes of interaction with the user interface can also be provided. For example, a gyromouse can be used, voice recognition can be used to allow for oral interaction with the user interface and an eye tracker can be used to track the positions of the surgeon's eyes so as to interact with the user interface. Various combinations of the interaction and input devices mentioned above can be used in different embodiments of the invention.

Camera part 104 of a tracking system includes a collapsable tripod 116 on which is mounted a head unit 118 including two mutually spaced apart infrared camera devices for capturing stereographic images of marker arrays (also referred to as stars or generally as fiducial markers). Head unit 118 also includes a source of infrared radiation which can be directed toward the marker arrays which bear infrared reflective spheres. By capturing images of the marker arrays, the position of marked entities (including body parts, instruments, implants) can be determined by data processing apparatus in the work station 102 so as to determine the position and orientation of the entities in the reference frame of the tracking system.

In alternate embodiments, other forms of tracking can be used. For example wire based tracking technologies can be used. Other forms of wireless tracking technology can include ultrasound or RF frequency electromagnetic radiation. An example of a suitable RF based wireless tracking system is disclosed in US patent publication US 2003/0120150 A1 (US patent application serial number 10/029,473, filed 21 December 2001)

Although not illustrated in Figure 1, workstation 102 can be wirelessly or wire connected to a computer network.

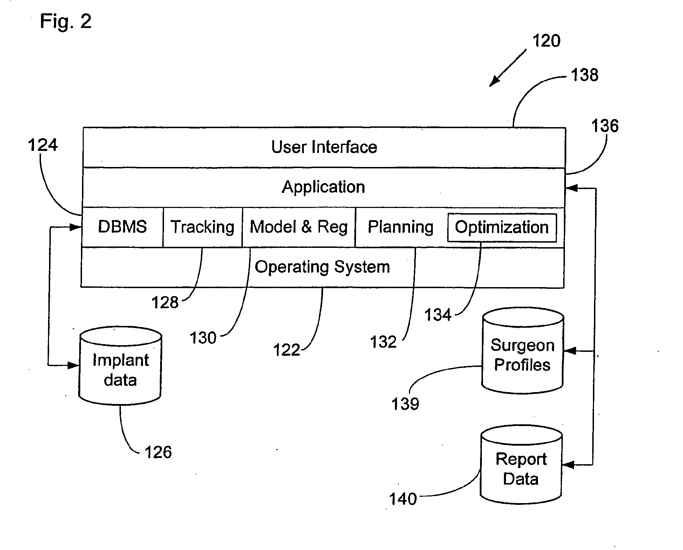

Figure 2 shows a schematic illustration of the software architecture 120 of the workstation 102. The computing device in workstation 102 includes an operating system 122. A number of modules are provided which interact with operating system 122. These modules can include a database management system 124 which can control a database 126 of implant data. Database 126 includes image files of implants and attributes of the implants which can be used with the CAS system. Implant database 126 also stores information relating to the geometry and shapes of the implants together with some default planning data for the implants as well as 3D images of tools and instruments that can be used with the implants.

A tracking module 128 is also provided which handles tracking operations and provides data identifying the entities being tracked and their current position and orientation in the reference frame of the tracking system. A statistical shaped modelling and registration module 130 is also provided to provide for registration of virtual models of the patient's body parts in the reference frame of the tracking system using statistical shape modelling techniques, as are understood in the art. A planning module 132 is also provided to allow surgical plans for the positioning of implants to be created. The planning module can include an optimisation process 134 which can be used to optimise the planned position of an implant. The functions provided by these modules are generally generic.

An application layer 136 sits on top of the modules and interfaces with the modules via application program interfaces in order to utilise the functions provided by these modules. The CAS application 136 defines various workflows which can be used by the surgeon to carry out various surgical procedures and defines the steps involved throughout the workflow at various stages, such as during planning, registration and navigated surgery. The CAS application 136 is in communication with a first database 139 storing surgeon profile data and can also communicate with a second database 140 storing report data. Databases 139, 140 can be local to workstation 102 or may be remotely accessible over a network. Preferably the surgeon profile data is local and the report data database is provided on a network storage device being part of an information system of a hospital in which the CAS system is located or to which the case system is connected over a network or via a communication link.

The user interface 138 of the present invention sits on top of the CAS application and provides the mechanism by which the surgeon can interact with the CAS system and control its operation.

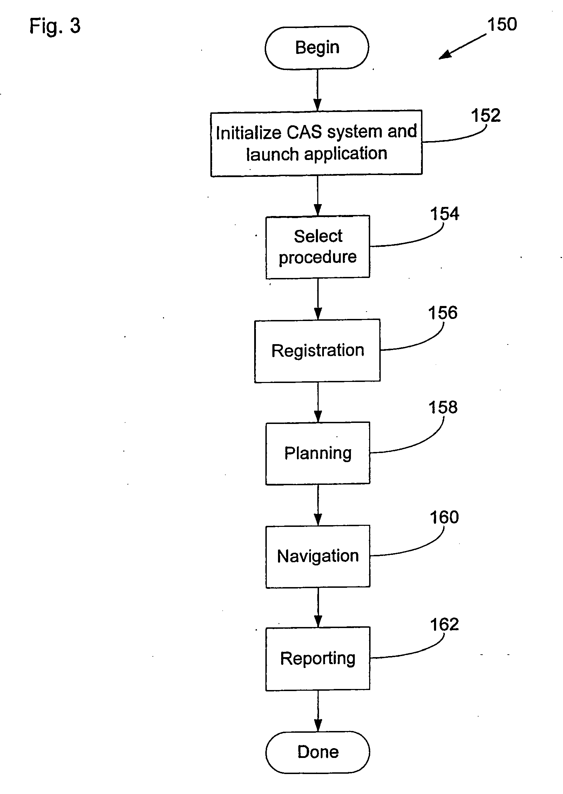

Figure 3 shows a flowchart illustrating, at a high level, the general method of use of CAS system 100 by a surgeon via the user interface of the present invention.

Method 150 begins and at step 152 the CAS system is initialised and the CAS application 136 is launched. The general steps involved in using the CAS system 100 include selecting the surgical procedure that is to be carried out 154, registering the body parts of the patient with the CAS system 156, planning the implant sizes and positions 158, carrying out a navigated surgical procedure 160 and finally preparing a report on the surgical procedure 162 incorporating data on how the surgical procedure is actually carried out. It will be appreciated that Figure 3 is schematic only and that some of these steps may be optional, e.g. reporting step 162, and that others of these steps may overlap to some extent, such as registration and planning, depending on the exact workflow of the surgical procedure being carried out.

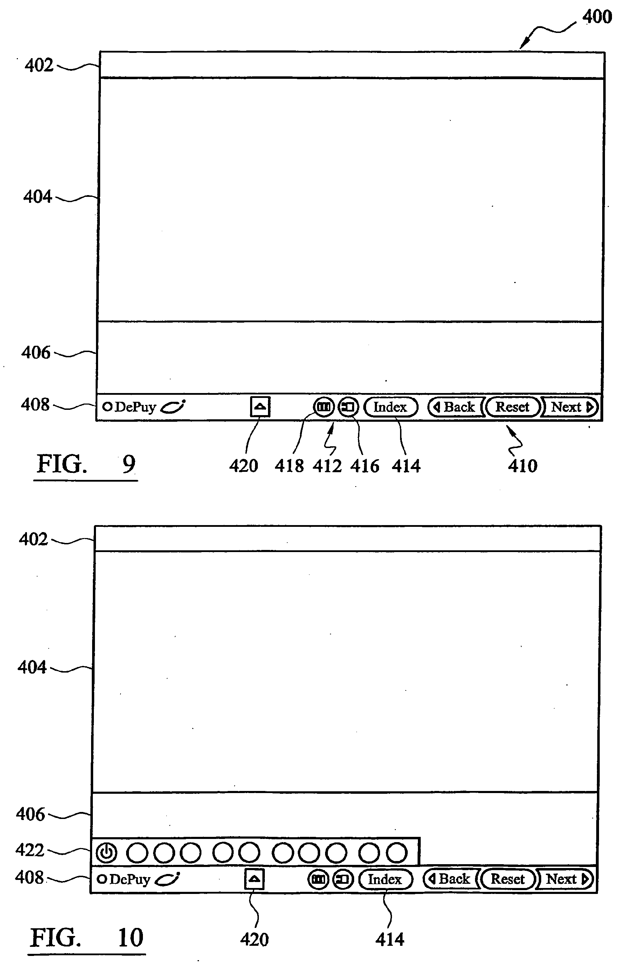

Before describing how the user interface is used to control the CAS system according to the method illustrated in Figure 3, the overall layout of the user interface during the registration, planning and navigation stages of the method will be described with references to Figures 9 and 10.

Figure 9 shows a screen shot of a screen 400 of user interface 138 illustrating the general layout of the user interface during the active stages of the CAS system, i.e. when it is actually being used in relation to surgical preparations, rather than setting up the system or reporting the results of surgery. The user interface includes a header portion 402 at the top and extending across the user interface. Below the header 402 there is provided a working or image display portion 404 extending across the user interface and having a generally constant height. Below the working portion there is provided an information portion 406 having a generally constant height and extending entirely across the user interface. Below the information portion and at the bottom of the user interface a control bar 408 is provided which also extends across the entire width of the user interface. It has been found that by separating the user interface into distinct portions having a certain type of information or control element located consistently therein, that it is easier to identify the most currently relevant information and to quickly and efficiently interact with the user interface to control the surgical procedure.

Header 402 can include text indicating the current stage of the current surgical procedure and also an indication of the proportion of which the current sub-stage of the procedure has been completed. Header 402 can also include an indication of the current surgeon and the patient on which the procedure is being, or will be carried out. Header 402 can also include a tracking status indicator which provides a visual indication of the tracking status as will be described in greater detail below.

Images of patient body parts are displayed exclusively in working portion 404 which may also include widgets or other control elements. Images can be presented in two different display modes as will be described in greater detail below.

Information portion 406 allows ancillary information to be displayed to the surgeon. Control elements or widgets may also be provided in information portion 406.

Control bar 408 includes control elements for navigating between screens 410 as well as a group of other control elements 412 which can be operated to control certain core functions of the user interface which are common to the different stages of the surgical procedure.

Navigation control elements 410 include a back button for returning to the previous screen, a next button for proceeding to the next screen and a reset button for resetting the current screen to its initial state.

Control bar control elements 412 include a button 414 displaying an index function, a button 416 for selecting a first display mode (referred to herein as "one up"), a button for selecting a second display mode (referred to herein as "three up") and a button 420 for displaying a toolbar 422 as illustrated in Figure 10. Toolbar 422 overlays a region of information portion 406 and includes a plurality of buttons which can be operated to access various tools provided by the user interface. The buttons on the toolbar 422 can be operated to provide a number of functions.

A button can be provided to activate a guidance feature. A button can be provided to activate a loud speaker. A button can be provided to capture an image. Respective buttons can be provided to increase magnification of displayed images and decrease magnification of displayed images. A button can be provided to select whether to use a morphed image of the body part or not. With this button the user can select, during a registration stage described in greater detail below, whether to display only registration points collected from the patients body part or to display the registration points together with a morphed image of the patients body part, in which the image has been morphed based on the captured registration points from a generic image using a statistical shape modelling technique.

A button can also be provided allowing the user to select whether additional information should be displayed. For example, in a ligament balancing screen the button can be used to select whether to display information relating to various gaps and/or angles or other information useful in ligament balancing. By way of further example, in a planning screen the button can be used to select whether to display extra or additional planning parameters. A button can also be provided allowing the user to select to access a generic ligament balancing screen at any stage during the surgery, so that the user can carry out a ligament balancing procedure A button can also be provided to allow the user to select to access a screen for verifying a morphed model of the patients body part as is described in greater detail below. A button can also be provided to allow further information to be displayed, such as information about an implant or implants, information about a chosen workflow or information about the version of the CAS application or user interface. A button can also be provided to turn off the system or to close the application. Operating toolbar button 420 again hides toolbar 422.

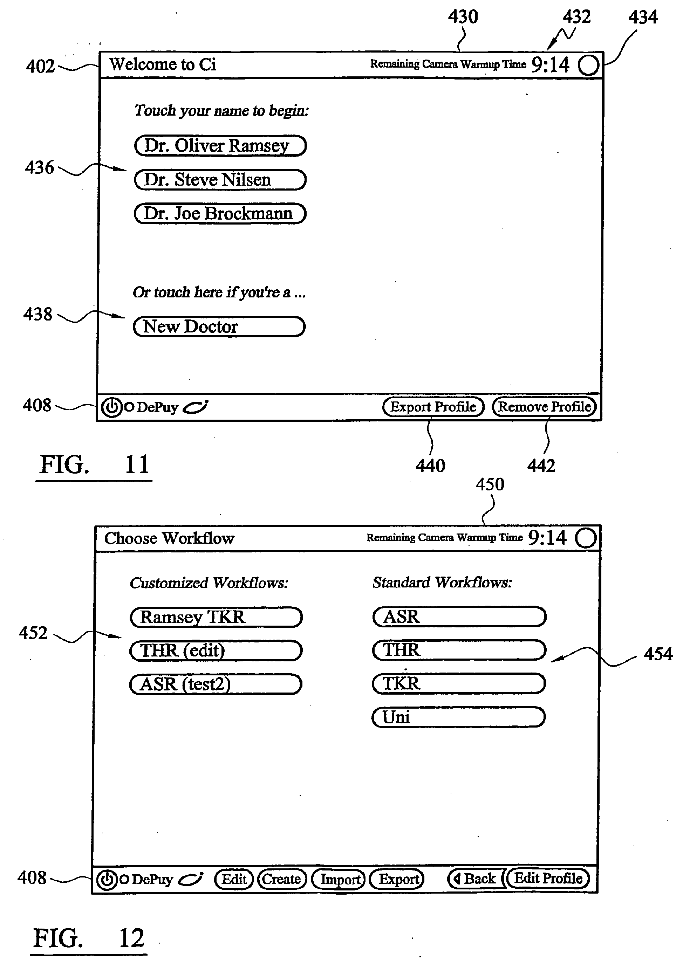

Referring back to Figure 3 and with reference to Figure 11, there is shown a screen shot 430 of an initial screen of the user interface which is displayed to the user after launch of the CAS application at step 152. Screen 430 is a first screen displayed by the user interface as a part of a procedure selection step 154 of the overall method. Header 402 includes a countdown timer 432 which indicates the amount of time remaining before the cameras of the tracking system can be considered to be up to working temperature. It has been found that leaving the cameras to warm up for 20 minutes provides improved tracking. Therefore, after initialisation of the CAS system at step 152 a timer is begun and the remaining time before the tracking system can be considered to be in an optimal working state is displayed so that the surgeon can delay beginning the procedure until the tracking system is optimal. Header 402 also includes a visual tracking status indicator 434 in the form of a coloured circle. Tracking status indicator 434 generally operates on a "traffic light" principle in which when all tracked elements are being reliably tracked the tracking indicator is coloured green. Should tracking of a one of the tracked entities fail, for example because there is an interruption in the line of sight between the cameras and the reference array, then the tracking status indicator changes colour, e.g. to red, to indicate that at least one of the entities is no longer being reliably tracked. During the camera warm up time, tracking status indicator 434 is coloured red to further indicate that the tracking system is not yet optimal.

Screen 430 includes three buttons bearing the names of three surgeons whose surgeon profiles are present in the surgeon profile database 138 of the CAS system. A surgeon profile includes various data items identifying the settings and other attributes used by the workflows of the CAS application as well as data identifying the surgeon and information relating to the surgeon. A further control element 438 is also provided in the form of a "new doctor" button which can be operated to allow a new surgeon profile to be created for the CAS system. Control bar 408 includes a control element in the form of an "export profile" button 440 which can be used to store a surgeon profile on a storage device. A further control element 442 in the form of a "remove profile" button is also provided and which can be operated to delete a surgeon profile from the CAS system.

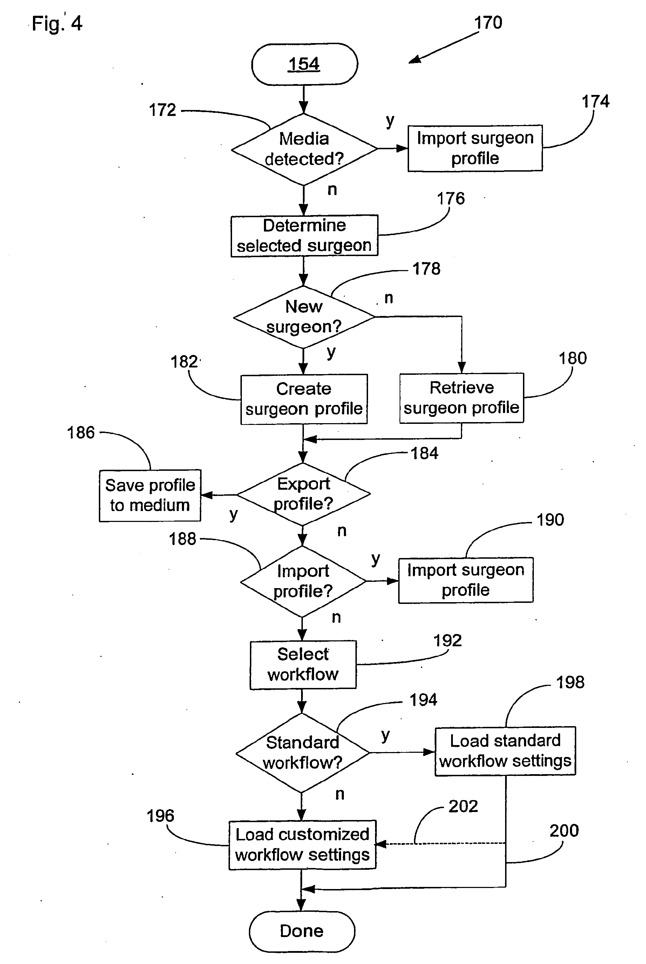

With reference to Figure 4 there is shown a process flowchart illustrating a process 170 by which a surgical procedure to be carried out using the CAS system is selected and corresponding generally to step 154 of method 150. The CAS system provides for the auto-detection and importation of a surgeon profile from a portable media device, such as a USB drive. This allows a surgeon to carry their surgeon profile with them so that they can use CAS systems at different locations using their surgeon profile even if their surgeon profile is not loaded on to the CAS system. Hence at step 172, CAS application 136 determines whether a data storage medium has been newly connected to the CAS system. If so, then at step 174 any surgeon profile present on the data storage device is imported and processing proceeds to step 192 at which screen 450 of the user interface as illustrated in Figure 12 is displayed.

If no data storage device is detected then processing proceeds to step 176 at which it is determined which surgeon has been selected by the user pressing one of buttons 436 or 438 of the user interface via the touch screen 112 of workstation 102.

At step 178 it is determined whether the selected button corresponds to a new surgeon and if not then at step 180 the surgeon profile for the selected surgeon is retrieved from database 138 and loaded into memory. Alternatively, a new surgeon profile is created at step 182 and the new surgeon is directed to enter various data items to set up their profile. The newly created surgeon profile is then stored in surgeon profile database 38.

At step 184, it is determined whether the export profile button 440 has been operated and if so then processing proceeds to step 186 at which the surgeon profile is stored to a storage device. The profile may be stored on a portable storage device, such as a USB drive for use on a different CAS system. At step 188 it can be determined whether the surgeon has selected to import a different profile. If so, then processing proceeds to step 190 at which a different surgeon profile for the currently selected surgeon is imported and loaded into memory. Alternatively, processing proceeds to step 192 at which the surgeon can select a workflow from the surgeon profile currently loaded into memory.

Figure 12 illustrates the screen 450 of the user interface displayed at step 192. Screen 450 includes control elements for selecting between three customised workflows 452 and four standard workflows 454. Control bar 408 includes a number of control elements which can be operated to edit, create, import or export an individual workflow, return back to the previous screen to select a different surgeon profile or to edit the current selected surgeon profile. Each customised workflow button 452 corresponds to a respective workflow which has been customised according to the particular preferences of the surgeon. Each of the four standard workflow buttons can be operated to select a standard workflow format but which nonetheless includes some default or customised data in order to configure the settings used during the standard workflow. The standard workflows a standardised protocol or sequence of steps wherein the surgeon can configure the data used at the different steps. Whereas the customised workflows the surgeon to change the protocol used, such as the type or sequence of steps, as well as the data used at each step in line with the surgeon's preferred practice.

At step 194 it is determined whether the surgeon has selected a customised workflow or a standard workflow. If a customised workflow is selected then processing proceeds to step 196 at which the settings for the selected customised workflow are loaded. Alternatively, if a standard workflow is selected then processing proceeds to step 198 at which the standard workflow settings are loaded into memory. For an existing surgeon, once the standard workflow settings have been loaded processing proceeds as indicated by line 200. However, for a new surgeon, some configuration of the settings for a standard workflow is required and so processing proceeds as illustrated by line 202 to allow some customisation of the settings for the standard workflow.

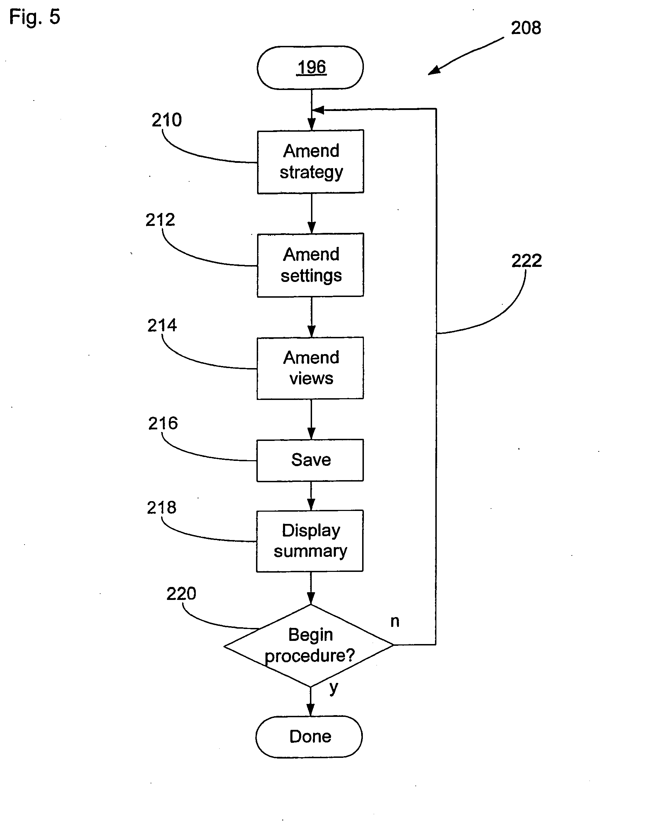

Figure 5 shows a process flowchart illustrating a customisation process 208 by which a surgeon can customise a workflow. Although described with reference to a custom workflow, a simpler version of process 208 is used to configure standard workflow settings for a new surgeon. Process 208 can be entered when a customised workflow is loaded or alternatively can be entered when creation or editing of a customised workflow is initiated from screen 450.

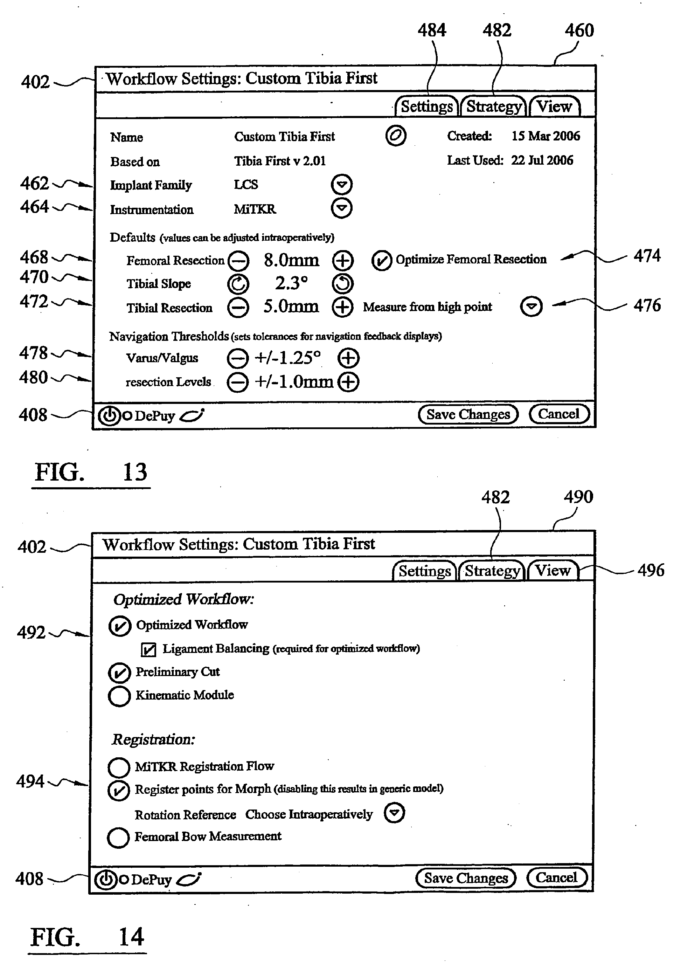

Workflow customisation process 208 begins with the option to amend the general strategy of the workflow at step 210. The surgeon can optionally select to amend details of the overall strategy of the workflow at step 210 by selecting strategy tab 482. On selecting strategy tab 482, screen 490 of the user interface as illustrated in Figure 14 is displayed. A group of control elements 492 is provided which the surgeon can select to customise the overall structure of the workflow for example by selecting whether to use an optimised workflow, whether to include ligament balancing, whether to include a preliminary cut and whether to use a cinematic module to assess the orthopaedic performance of the planned implant positions. A further group of control elements 494 is also provided to allow the surgeon to customise the registration process. This can include selecting whether to use a minimally invasive registration workflow, whether to capture bone points for morphing a bone model, how to select a rotation reference and whether to carry out a femoral bow measurement.

By selecting tab 484, the user can optionally select to amend various settings used during the workflow. Figure 13 shows a screen 460 of the user interface displayed at step 212. Header 402 displays the title of the workflow being customised. A variety of attributes of the workflow and data items used during the procedure defined by the workflow can be mended or edited by the surgeon. For example, a control element 462 in provided to allow the surgeon to identify the implant family to be used in the procedure. A further control element 464 is provided to allow the surgeon to select the instrumentation to use in the workflow. A number of control elements are also provided to input default planning data for the surgical procedure. In this instance, the surgical procedure is a total knee replacement arthroplasty in which the tibial cut is carried out first. A control element 468 is provided to allow the depth of the femoral resection to be entered using increment and decrement buttons and also displaying the selected femoral resection depth. Similarly a control element 470 is provided to set a value for the tibial slope which includes buttons to increase and decrease the slope and also a display of the selected slope value. A further control element 472 is provided to set the tibial resection height. A further control element 474 is provided to allow the surgeon to select whether an implant optimization routine should be used during the planning process. A further control element 476 is provided to allow the surgeon to determine the point from which the tibial resection depth is measured.

Further control elements 478, 480 are also provided to allow the surgeon to set threshold values for the navigation tolerances displayed during subsequent navigation screens. These values are used to provide a visual indication of whether the navigator position of an entity is sufficiently close to the planned position as will be described in greater detail below.

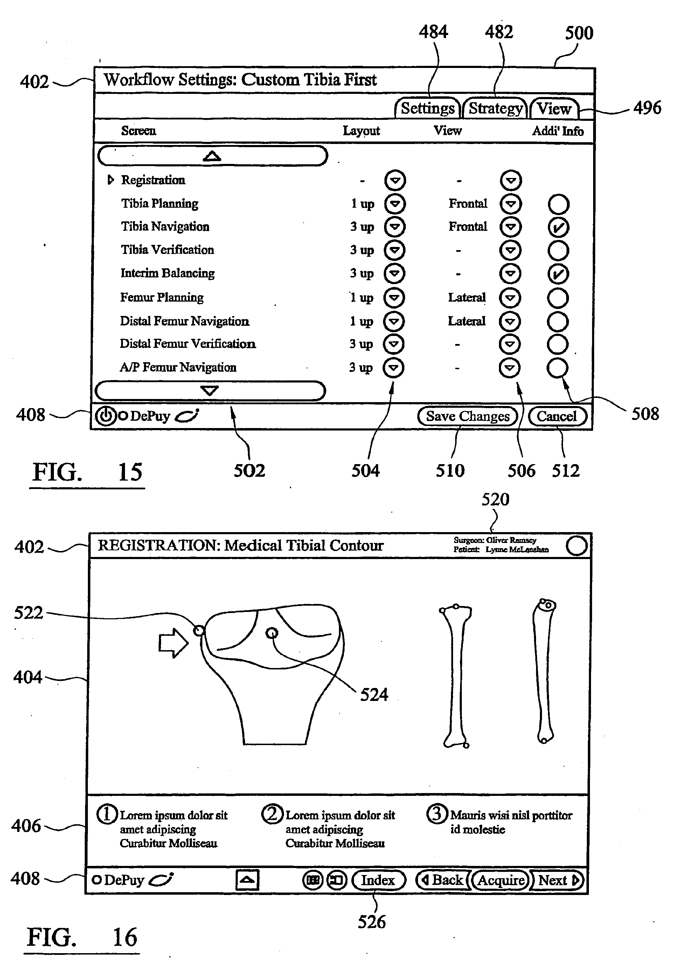

After any amendments to the strategy and/or settings have been made, then optionally processing can proceed to step 214 to customise the views of the images displayed at various stages throughout the procedure. On selecting view tab 496, screen 500 of the user interface as illustrated in Figure 15 is displayed. View customisation screen 500 includes a first column 502 which lists each of the screens displayed during the surgery associated steps of the procedure. A second column 504 provides a control element which allows the surgeon to select the layout of images to be displayed for each screen. A third column 506 is provided which includes a control element allowing the direction of view of the images in the selected layout for each screen to be selected. A fourth column is provided 508 of control elements allowing the surgeon to select whether additional information should be displayed for each of the screens.

The user can select to swap between the settings, strategy and view screens by selecting tabs 482, 484 and 496. The surgeon can select to save the customised workflow at step 216 by operating save changes button 510.

In an alternative embodiment, a different approach can be used to customise the workflow. Instead of having separate settings, strategy and view screens, a sequence of screens for a dummy generic workflow for the procedure are sequentially displayed to the user. As the user progresses through the screens, the user can enter commands and data to select the various settings, strategy and view properties described above as appropriate for each screen in the workflow. Then at the end of the workflow, the workflow so customised by the user is saved at step 216 to provide the customised workflow.

At step 218 a summary of the customised procedure can be displayed to the surgeon. At step 220, the surgeon can enter a command indicating whether to begin the procedure with the customised workflow or alternatively, as illustrated by line 222, to return to the customisation screens to amend the workflow. When the surgeon is happy with the customised workflow then the surgical aspects of the method can begin.

Referring back to Figure 3 now that the workflow for the procedure has been prepared, the method can move on to the surgical steps of the method, i.e. those associated with actually carrying out the surgery rather than setting up the CAS system to carry out the surgical procedure. In the following, the registration, planning and navigation steps are generally referred to as surgical steps although it will be appreciated that it is substantially the navigation steps which actually relate to performing the surgical procedure. However, the registration and planning parts of the method are also referred to as "surgical" herein as they relate to activities carried out under control of the surgical workflow, rather than merely to prepare the surgical workflow.

At step 156, registration activities can be carried out. Registration generally refers to registering an image representing the patient's body part in the reference frame of the tracking system which tracks the positions of the implants, instruments and tools used by the surgeon to carry out the surgical procedure. Depending on the workflow, registration may be carried out in an initial single step or various registration steps may be required throughout the surgical procedure. For example in a standard total knee procedure both the femur and the tibia may be registered. In contrast, in a minimally invasive tibia first workflow, initially the tibia is registered and then the tibial cut planned and executed before the femur is registered and then the femoral implants planned and femoral cuts made. Therefore, the separation of registration, planning and navigation in Figure 3 is conceptual only and is not intended to limit the invention to isolated separated procedures.

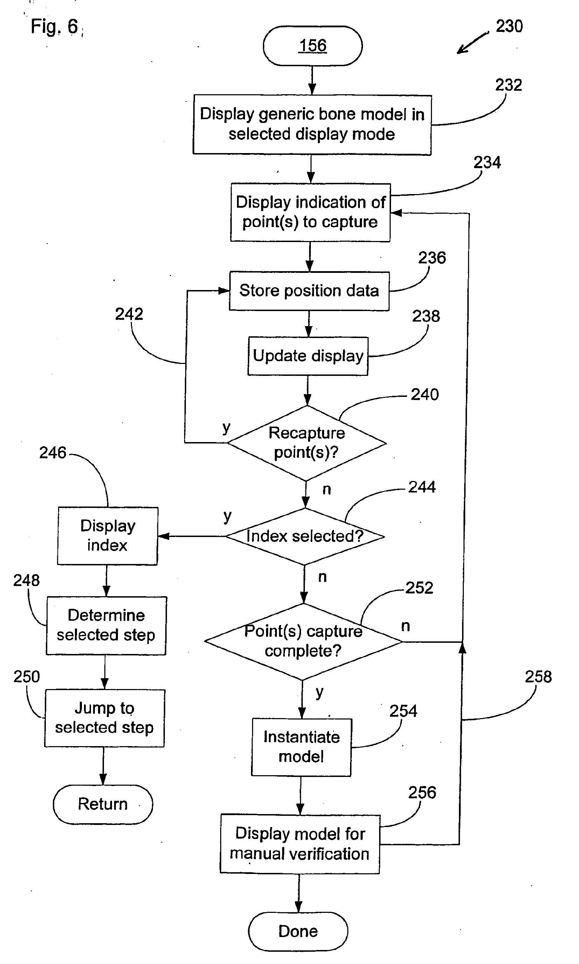

With reference to Figure 6, there is shown a process flowchart illustrating a registration process 230 by way of illustration of how registration can be carried out using the interface of the current invention. Figure 16 shows a screen shot 520 of the user interface via which the surgeon interacts with process 230. In the illustrated embodiment, registration of the tibia is being carried out although this is by way of example only.

In the described embodiment, registration is based on a statistical shaped model methodology as is generally known in the art. In a statistical shaped model approach, a generic model of an "average" bone is initially created. Information relating to the actual shape and size of the patient's bone is collected using a trackable pointer and then the generic model is morphed to more closely resemble the patient's actual bone shape and size. As the positions of the actual bone of the patient are determined in the reference frame of the tracking system, the position of the morphed model of the patient's bone is automatically registered in the reference frame of the tracking system.

At step 232, the registration process begins by displaying an image of the generic bone model in the working portion 404 in the user interface in the display mode selected and with the particular views selected during customisation of the workflow. As illustrated in Figure 16, three images of different views of the tibia are displayed in a "three up" mode in which each image has the same size, i.e. has the same height within the working portion, although the proximal part of the tibia is shown at greater magnification. At step 234, an indication of the registration points to be captured using a tracked pointer is displayed. The user interface highlights the registration point to be captured with an arrow and also with a coloured dot 522. Registration points which have not yet been captured are also displayed in the user interface by coloured dots, e.g. 524, but in a different colour to that which is to be captured. For example, the point to be captured may be coloured green and the points that have not yet been captured may be coloured red. At step 236 the position of the registration point is determined from the position of the marked pointer as tracked by the tracking system and stored. At step 238 the display is updated to reflect that the current registration point has now been captured and the colour of the registration point is changed from green to red. At any stage in the digitisation of registration points, the user can select to recapture a registration point, as illustrated by decision box 240. The user can select the registration point to recapture by touching the point on the user interface via the touch sensitive screen and then applying the tracked pointer to the corresponding point on the patient's bone. The position of the recaptured registration point is then stored as illustrated by process flow line 242.

At any stage during the registration procedure, or indeed during screens for any other steps of the method 150, an index function can be selected by operating index button 526 in control bar 408 in order to allow the user to jump to a specific screen of the user interface.

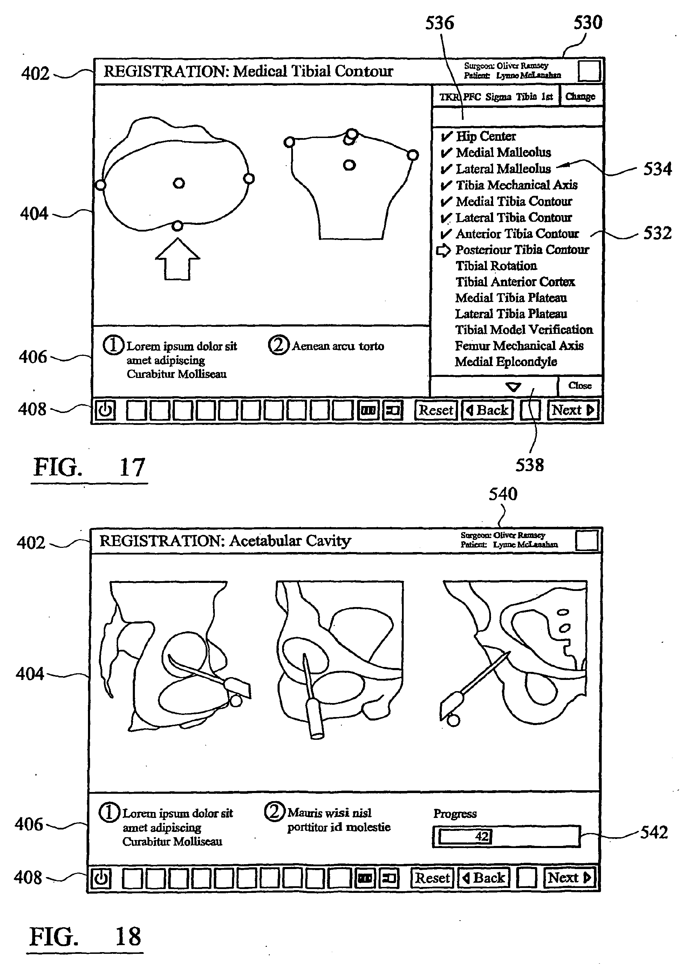

Figure 17 shows a screen shot 530 of a registration screen of the user interface after the index button has been operated. As illustrated by decision box 244, if the index function is selected at any time, then processing proceeds to step 246 at which the index is overlayed on the user interface so as to display the index. The index 532 comprises a sequential list of the screens that are displayed by the user interface according to the selected workflow. The index lists the screens by title 534 and provides an indication of those screens which have already been navigated through by a tick and an indication of the screen currently being accessed by an arrow. Screens which have not yet been accessed are un-ticked. Control elements are provided toward the top 536 and bottom 538 of the list which can be operated to allow the user to scroll through the list. If the surgeon decides that they want to return to a previous screen, then rather than using the back control element, the surgeon selects the screen to jump to by touching the title of the screen. The corresponding screen is then displayed and the index is removed.

The surgeon can also select to jump forward to a screen which has not yet been traversed in a similar manner. However, the index is an intelligent index and does not permit the user to jump to screens which cannot yet be used because a preceding part of the workflow has not yet been carried out and is required in order for the later part of the workflow to be carried out. For example, the index can prevent the surgeon from jumping ahead to a navigated surgery part of the workflow if registration has not yet been carried out. Those screens which correspond to parts of the workflow which cannot currently be jumped to can be rendered inactive until the preceding steps required for them to be carried out have themselves been completed. The screens of the workflow which cannot yet be jumped to can be displayed in a visually distinct manner, for example by being greyed out, displayed more faintly or in a different colour, so that the user can readily identify screens which are not yet active. The surgeon can select to close the index without jumping screens.

Also, the index can include an indication of screens which will not be accessed by the current workflow but which the surgeon can select to include in the workflow and access via the index. For example, a ligament balancing procedure may be omitted in the selected workflow. However, the ligament balancing screen is displayed in the list in a contrasting fashion, e.g. by being greyed out. If, during the procedure, the surgeon decides they do want to carry out ligament balancing, then the surgeon can touch the ligament balancing screen title in the index and that screen is included in the workflow and the user interface immediately jumps to the corresponding screen. Hence, the index provides an easy manner for the surgeon to navigate immediately to a screen of choice rather than passing sequentially through screens. It also provides a mechanism allowing the surgeon to change the workflow on the fly.

As illustrated in Figure 6, when the surgeon touches the screen title to jump to, at step 248 the application determines which step of the procedure has been selected and at step 250 jumps to the selected step so that the corresponding screen of the user interface is displayed to the surgeon. Process control then returns to the application at the point corresponding to the displayed screen.

Returning to the main flow of the process, at step 252 it is determined whether all the points required for registration have been captured. This determination may be automatic, because there are a finite number of registration points which the workflow forces the surgeon to collect or alternatively the surgeon may enter a command to indicate that they have collected those registration points that they want to collect in order to carry out registration. If the required registration points have not yet all been captured then processing returns to step 234 at which the display is updated to indicate the next registration point or points to capture.

Figure 18 illustrates a further registration screen 540 of the user interface illustrating digitisation of the acetabulum of the patient's pelvis. As illustrated in Figure 18, a three up display mode is being used to illustrate three different views of an image of a pelvis together with an image of the pointer being used to digitise the patient's actual pelvis. In order to register the acetabulum, a cloud of points over the surface of the patient's acetabulum is collected by running a marked pointer over the surface of the patient's acetabulum and the tracking system captures the position of a large number of points. The user interface includes a progress metre 542 which indicates the proportion of the points required for registration which have been captured. The progress metre is in the form of linear bar gauge in which the length of the bar represents the proportion of the total number of points which have currently been captured together with a numerical indication of the percentage of points which have been captured. As illustrated in Figure 18, 42% of the points required for complete registration have been captured. This provides a visual indication to the surgeon as to how many further data points need to be captured so that the surgeon can ensure that the cloud of points is collected equally over the entire surface of the acetabulum to ensure an accurate morphing of the model pelvis. In this example, the CAS application automatically determines when sufficient points have been captured at which stage the capture of points is automatically terminated.

Returning to Figure 6, once all the points required for registration have been captured, processing proceeds to step 254 at which a model of the patient's body part is instantiated from the statistical shaped model data points in order to morph the generic model into a closer representation of the patient's actual anatomy.

The surgeon can select to re-capture any one of the registration points if he considers the point to be inaccurately captured or to be causing a poor registration. The surgeon can simply select the point to be recaptured by touching the registration point on the screen. The user interface then jumps to the screen corresponding to capture of that point. The position of that point is then recaptured and the user interface then jumps directly back to the registration step to register the body part using the newly recaptured registration point position with the original other registration point positions. Hence, the surgeon does not need to recapture all the other, or some of the other, registration points in order to re-do the registration as would be required by a completely linear registration work flow.

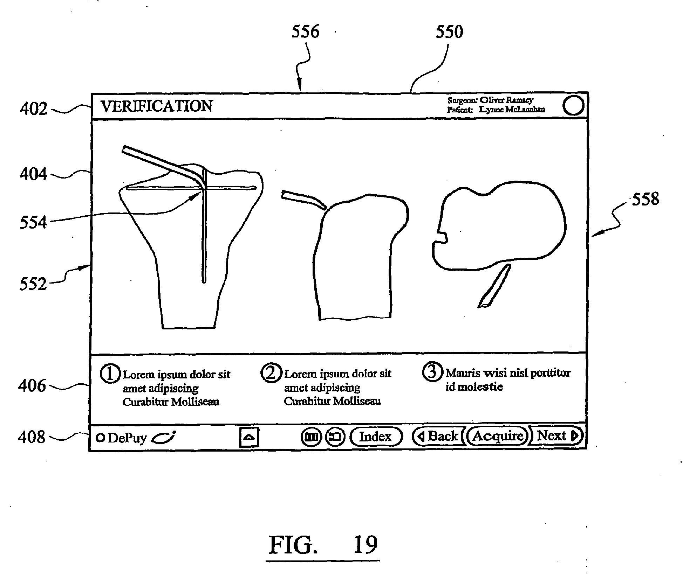

At step 256 the instantiated model of the patient's anatomy can optionally be displayed for manual verification. Figure 19 shows a screen shot 550, of the user interface at verification step 256. The user interface displays images of the model each of the same size, in three up mode. A first image 552 shows an image of the surface of the model together with an image of the current position of a pointer touching the corresponding position of the patient's actual bone. A cross hair target 554 is overlayed on the image of the bone centred on the position of the pointer tip. A second image 556 in the working portion shows the cross section through the instantiated bone model along the vertical cross hair together with an image of the tip of the pointer. A third image 558 in the working portion shows a cross section through the instantiated bone model along the horizontal cross hair together with an image of the pointer. Additionally, or alternatively, the one, or each, of the cross sectional views 556, 558 can include an indication of the other plane, for example, the sagittal cross section 556 can also include an indication of the position of the transverse plane and the transverse cross section 558 can include an indication of the position of the sagittal plane.

The cross hair target helps the surgeon to identify the planes through the bone to which the cross sections correspond. This can be further enhanced by providing colour coding between the target and the cross sections. For example the vertical cross hair can be displayed in a first colour, e.g. green and the outline of the corresponding cross section can be displayed in the same colour. The second cross hair can be displayed in a different colour, e.g. red, and the outline of the corresponding cross section can be displayed in the same colour. This provides an immediate visual clue as to which cross section corresponds to which orientation of the bone model.

Hence, the displayed images allow the surgeon to verify the accuracy of the instantiated bone model. The surgeon can run the tip of the tracked pointer over the patient's actual bone and view the differences between the position of the pointer on the screen and the position of the surface of the bone model to verify how accurate the instantiation is. If the surgeon determines that the instantiated model is not acceptable then processing flow can return, as illustrated by line 258, and either further registration points can be collected or previously captured registration points can be recaptured as described above.

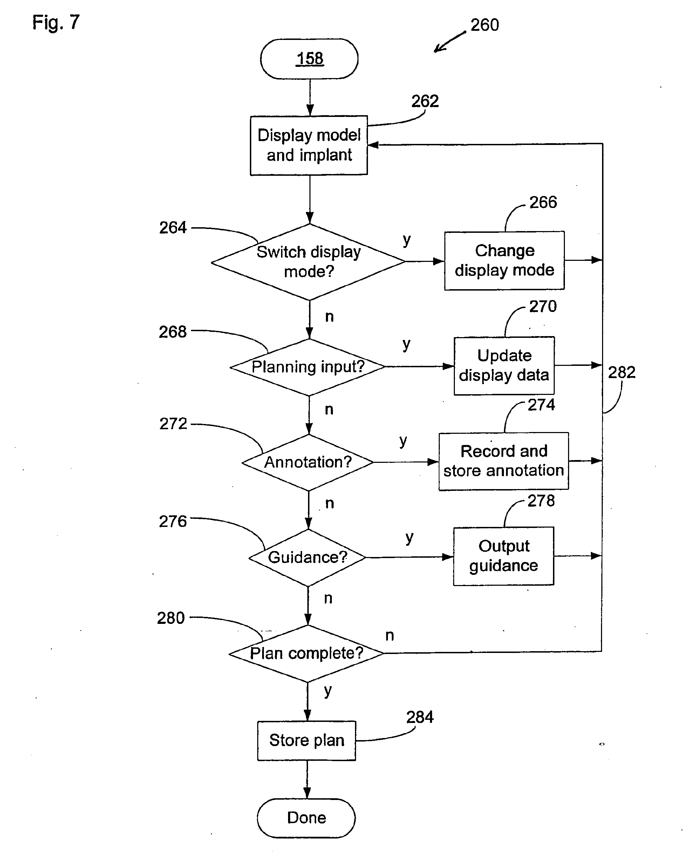

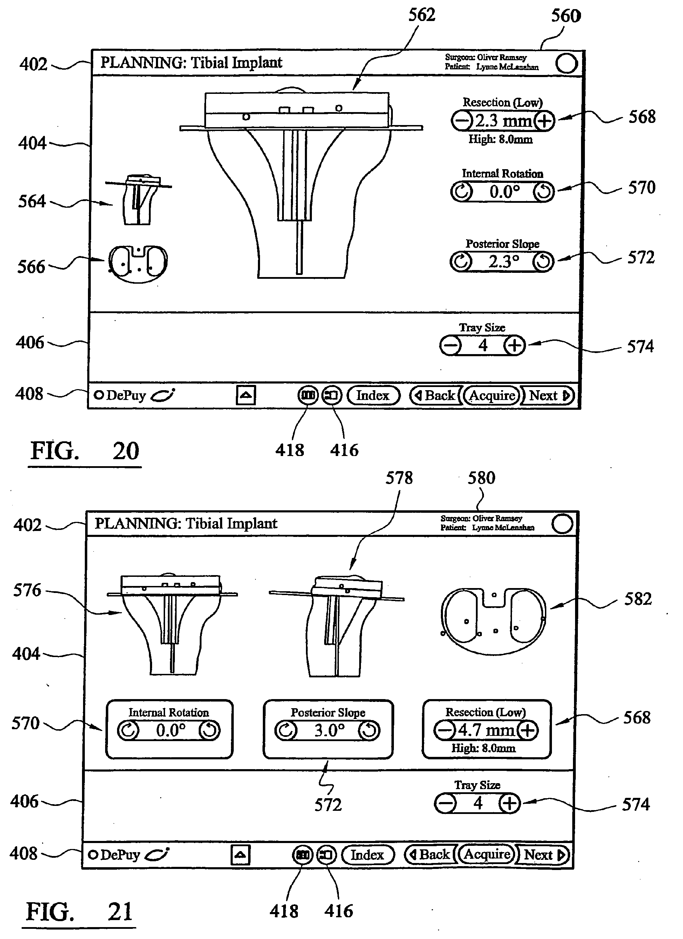

With reference to Figure 7, there is shown a process flowchart illustrating a planning process 260 corresponding generally to operations 158 of method 150. The planning process 260 is by way of illustration of the various functionalities supported by the user interface in order to carry out planning, but is not limited to the specific planning operations shown or described in the accompanying screen shots. The planning process 260 begins and initially the bone model and implant are displayed in the user interface. Figure 20 shows a screen shot 560 of the user interface at step 262 illustrating a first display modality. In this first display modality, also referred to herein as "one up", a primary image 562 is displayed in the working portion of the user interface together with a second smaller image 564 showing a different view and a third image 566 also smaller than the main image 562, showing a further different view. Second and third images 564 , 566 are presented as thumb nails showing different alternate views to the main image 562. The surgeon can select to display a one of the thumb nail views as the main image 562 by touching the thumb nail. The thumb nail view is then displayed as the major image 562 and the previous major image is displayed as a thumb nail image. Hence, the surgeon can select which view to display as the main image in the working portion 404 while still having easy access to other views which can easily be interchanged.

The user interface includes three control elements, or widgets, for altering the planned position of the implant 568, 570, 572 and one control element or widget for selecting the size of the implant 574. Each widget includes a first button for incrementing the value of the associated property, e.g. resection, height, internal rotation or posterior slope, and a second control element for decrementing the value of the associated property. The control element also includes an indication of the current value of the property and also text describing the planning property. Similarly, the implant size control element 574 includes control elements for incrementing and decrementing the implant size, a display of the current value of the implant size and text identifying the implant.

In the first display modality illustrated in Figure 20, the planning control elements 568, 570, 572 are all disposed toward the right-hand side of the display portion of the user interface. The implant size control element 574 is located in the information portion 406 and also toward the right-hand side of the user interface. It should be noted that none of the control elements are interspersed within the portion of the working area 404 in which images are displayed. Rather, the control elements and image are segregated so as to make it easier for the user to identify the visual information of immediate relevance and also being able to easily locate and operate control elements.

In one embodiment the image of the different views of the bone are static. However, in another embodiment, the image of the bone can be rotatable by a user operating a control element to rotate the bone about various axes, or alternatively, dragging a cursor over the image using a pointing device to cause the image of the bone to rotate.

Figure 21 shows a screen shot 580 of the user interface corresponding substantially to screen shot 560 in Figure 20 but in which the working portion 404 is configured in second display modality. In the second display modality three images of different views of the bone model and implant are displayed 576, 578, 582 which each image being of the same size. In this second display modality, sometimes referred to herein as "three up", the surgeon is presented with images of different views of the implant and bone model in which each image is of the same size.

Control elements 568, 570 and 572 are located toward the lower part of working portion 404 but again the control elements are segregated from the images such that a region of working portion 404 displays images only and does not include control elements. It is preferred if the control elements are associated with the image in which the effect of the control element can most clearly be illustrated. For example control element 572 which allows the posterior slope angle to be selected is associated with image 578 which most clearly illustrates the angle of the posterior slope.

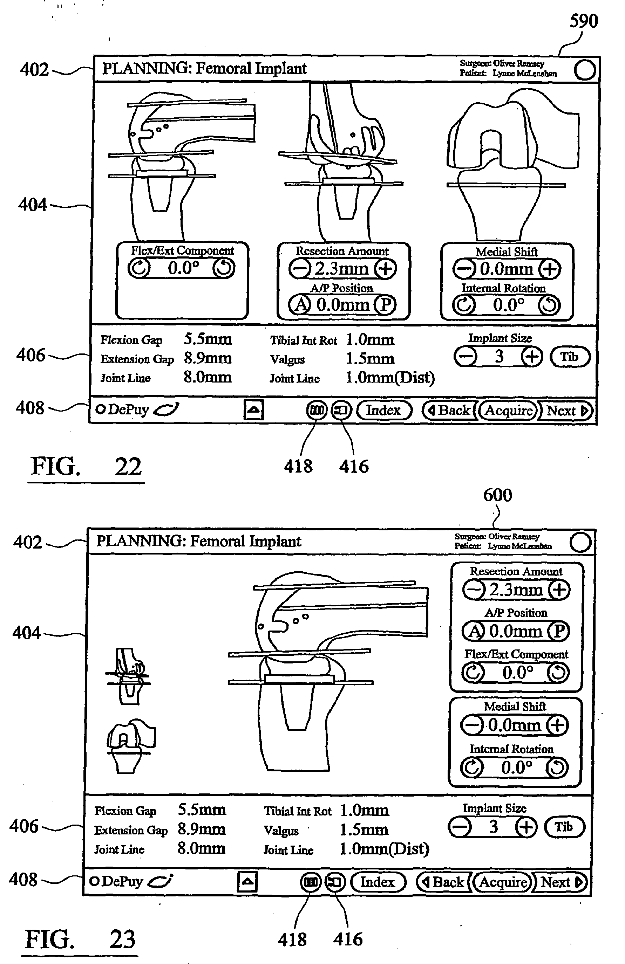

Figures 22 and 23 respectively show screen shots 590, 600 of the user interface for the same planning stage for a femoral implant but in three up and one up display modes respectively. As illustrated in Figure 22, in the three up mode different views of the patient's bones and the implants at their planned positions, each of the same size, are displayed in the working portion 404. Segregated from the images are control elements similar to those shown in Figures 20 and 21 via which the surgeon can enter position and orientation data in order to plan the position of the femoral component. Figure 23 shows the same images and control elements, but with the working portion 404 configured in the one up display mode with the control elements arranged separately to the images toward the right-hand side of the user interface. It will be appreciated that in other embodiments, the control elements can be disposed toward the left-hand side of the images instead. Figures 22 and 23 also illustrate the provision of further information pertaining to the images shown in the working portion in the information portion 406. Although the planning screen illustrated in Figures 22 and 23 has been described after the planning screen illustrated in Figures 20 and 21 and before navigation, it will be appreciated that depending on the workflow, these planning screens need not follow immediately after tibial planning and, for example in a tibia first workflow, may follow after navigated tibial cut and registration of the femur.

Returning to Figure 7, at any time, the surgeon can select to switch between the three up and one up display modes by selecting control elements 418 or 416 in the control bar. If it is determined at step 264 that the surgeon has entered a command to swap display modes, then the working portion of the user interface is reconfigured to display the appropriate images and reposition the control elements. As also described above, in the one up mode, the user can select a one of the thumb nails to form the main view and select which one to display by touching the image in the user interface.

At step 268, it is determined whether the user has entered planning information by operating any of the planning widgets, for example by pressing the plus or minus buttons, anterior or posterior buttons or clockwise or counter clockwise rotation buttons. If planing input is determined at step 268 then processing proceeds to step 270 at which the screen display is updated to display the new value for the implant position or orientation value and also the new position of the implant in the images.

At any stage in the procedure, and also at any time during the registration or navigation operations, the surgeon can select to record comments on the current step of the surgical procedure or otherwise include annotations explaining or describing the procedure. For example, the surgeon may want to record his reasons for planning a particular component position, orientation or size. A control element can be operated to select to record the surgeon's comments and the surgeon can speak the comments which are recorded by a microphone and stored as an audio file. Alternatively, the comments or annotations can be recorded by entering text using a soft keyboard, hardware keyboard or any other input device. Hence at step 272 if it is determined that the surgeon does wish to add comments, then processing proceeds to step 274 at which the comments are recorded and stored for further use. Images associated with the comments can also be selected and stored.

At any time during the process the surgeon can select a guidance feature from the toolbar. The guidance feature is available at any stage of the overall process from all or any of the screens of the user interface. If it is determined at step 276 that guidance has been selected by the surgeon then at step 278 a context dependent guidance is output to assist the surgeon. The guidance is context sensitive in that guidance appropriate to the current step of the procedure is output when guidance is selected.

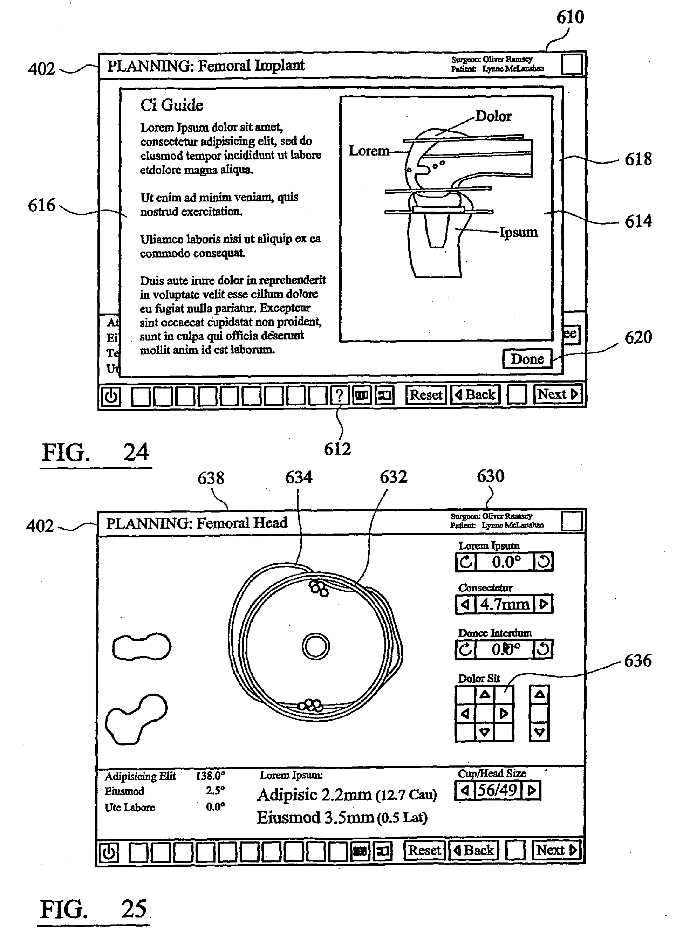

Figure 24 shows a screen shot of a screen 610 of the user interface illustrating the guidance feature which has been activated by pressing the "?" button of the toolbar 612. In the illustrated embodiment, the guidance takes the form of an image 614 related to the planning step currently being carried out together with text 616 explaining the current planning step. The window 618 including the guidance information is overlayed over the current planning screen and can be closed by pressing "done" button 620.

Various media types can be used to provide the guidance. Guidance can include a sequence of images, an animation, video footage of an actual procedure, audio and any one or combination of these. Further, the guidance feature can search the stored annotations for the current workflow to determine whether any annotations for the current step have been stored either by the same surgeon or a different surgeon. Those annotations can also optionally be displayed as part of the guidance. Hence in this way, the expert knowledge of other surgeons can accessed by a surgeon to see how a potentially difficult issue was addressed previously. The guidance information can also include information from an expert system indicating the appropriate approach to take at any step thereby providing further support to the surgeon.

Figure 25 shows a screen shot of a further planning screen 630 of the user interface. This planning screen corresponds to the step of planning the position of the femoral head component in an articulate surface replacement surgical procedure. The main image of the one up display shows the current planned location of a femoral head implant component 632 and a cross section 634 through the femoral neck. The user interface screen includes a widget 636 for adjusting the position of the femoral head implant component 632 over a plane substantially perpendicular to the femoral neck access. Widget 636 includes four control elements, in the form of arrow buttons, which are arranged in a cruciform manner. By operating a one of the buttons, the position of the femoral head implant component can be translated over a plane.

A further feature illustrated by screen 630 is the provision of an indication of the progress of the procedure in header 402. Progress indicator 638 shows that the current screen is the third screen out of a total of 15 screens to be navigated in order to complete the current stage of the procedure, i.e. the planning stage. This provides indication to the surgeon of how much of the current stage of the process has been completed.

Returning to Figure 7, at step 280, if planning has not yet been completed then process flow returns to step 262, as illustrated by line 282. Line 282 also schematically illustrates that following any other interaction with the planning screens of the user interface, process flow returns to step 262 so that any of the activities described can be carried out in any sequence. When it has been determined at step 280 that the surgical plan has been completed then at step 284 the plan is stored and the planning stage of the process is complete.

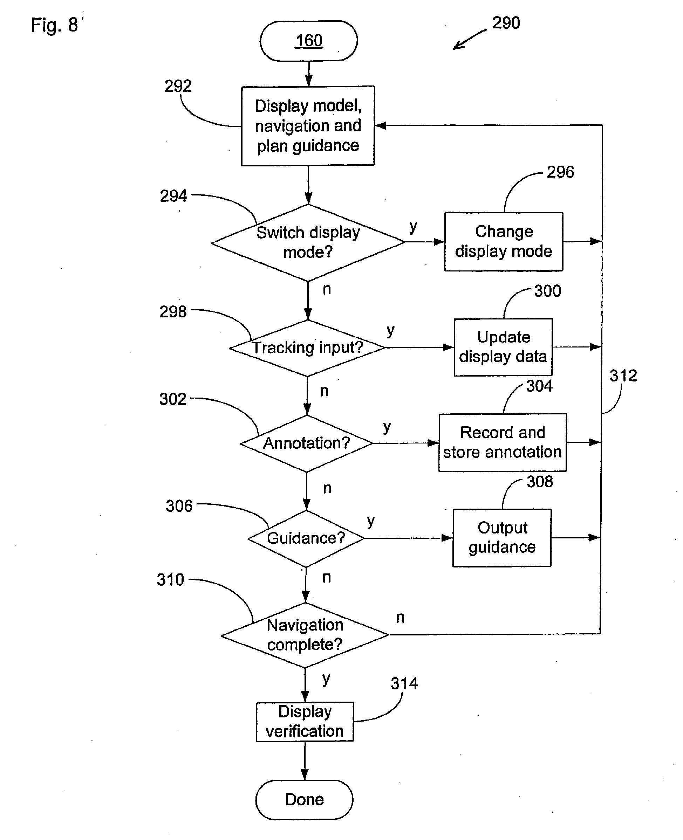

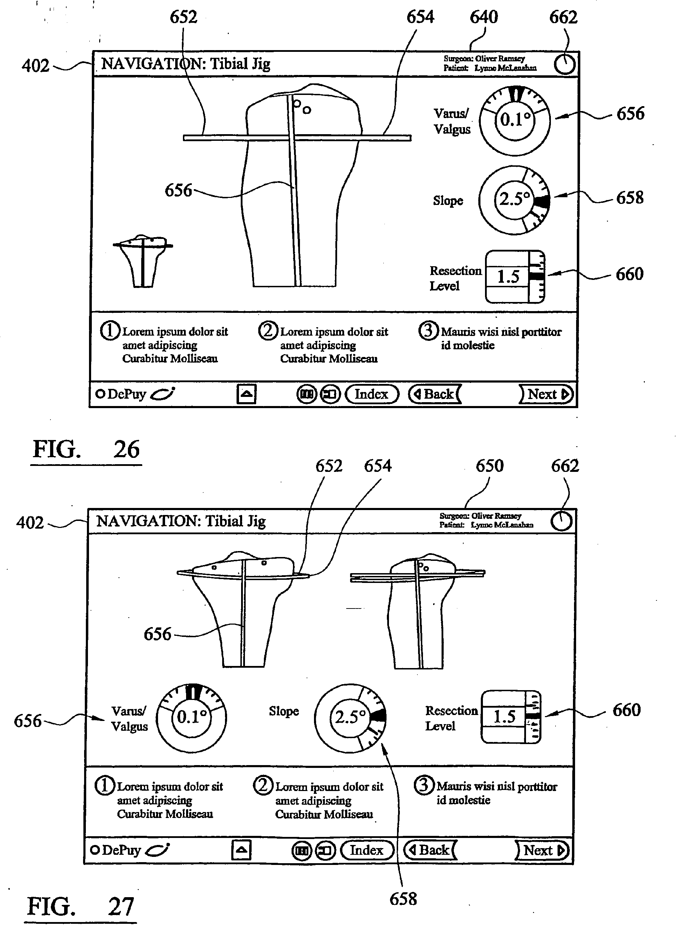

Figure 8 shows a process flowchart illustrating a navigation process 290 used during navigation steps 160 of the overall process. Figure 26 shows a screen shot of a screen 640 illustrating a tibial cutting jig navigation step. Figure 27 shows a screen shot of the same tibial jig navigation screen 650, but in the alternate display mode. For this particular screen, only two different views of the body part have been selected for display and so while the first display mode may still be referred to as the one up mode, the second display mode, illustrated in Figure 27, may more properly be referred to as a two up mode.

The navigation process 290 begins at step 292 at which images of the bone model, from different views, are displayed together with an indication of the position of the navigated item, in this instance a tibial cutting jig and an indication of the planned position for the current step of the procedure, in this instance the first tibial cut. In the illustrated embodiment, the indication of the navigated position is provided by a planar disc 652 displayed in a first colour and the planned position is illustrated by a second planar disc 654 in a second colour, different to the first colour. Also illustrated are the positions of the points captured during registration and an indication of the anatomical axis of the tibia 656.

In one embodiment, in the navigation screens, a schematic representation of any instrument or instruments used for the current workflow step can be displayed to the user in the user interface, for example in the information portion 406. The user can then select to display further information relating to the instrument or instruments, e.g. how to use the instrument, how to assemble or disassemble the instrument, by selecting the instrument, e.g. by touching the representation of the instrument on the touch screen. A pop-up window can then be displayed to the user including the information relating to the instrument.

A number of the steps illustrated in Figure 8 are the same as those described with reference to Figure 7 and so will not be described again. Figures 26 and 27 illustrate the different display modes which can be switched between at step 294 by the surgeon. At step 298, it is determined whether there is input from the tracking system, which reflects that if any of the entities displayed in the working area that have been tracked by the tracking system have moved. If so then at step 300, the display is updated to provide a real time indication of the position of the displayed entities and processing returns to step 292.

Screen 640, 650 includes three widgets providing a visual indication of the agreement between the navigated position of the tibial jig and the planned position 656, 658, 660. Each widget is in the general form of a metre including a graduated scale. The scale includes a portion in a first colour which indicates the planned value of the planning property, e.g. slope and the range of values which can be considered to be within tolerance of the planned value, e.g. plus or minus 0.1°. The widget also includes a pointer which indicates the current position of the tracked entity. The widget also displays the planned value for the planning property.

As the tracked entity is moved, the pointer for each of the widgets moves accordingly to reflect the current position and orientation of the tracked entity. When the tracked entity is within tolerance of the planned position for orientation then the pointer lies within the planned range of the scale, and the pointer changes colour and the background on which the planned value is displayed also changes colour to highlight that the particular property is now within tolerance of the planned value. For example when the slope of the tibial jig is greater than 2.6° or less than 2.4°, the pointer is coloured red and the circular background is displayed in red. However when the position of the jig provides a slope within 2.6° to 2.4°, the pointer turns green and the background circle turns green to show that this planning property is now within tolerance. Hence these widgets provide an easy to read graphical indication of whether the navigated entity is at the planned position and also an indication of how the navigated entity should be repositioned in order to correspond to the planned position by virtue of the moveable pointers.

Screen 640, 650 also includes a tracking status indicator 662 in the form of a circle whose colour and appearance can change in order to indicate whether all required entities are being successfully tracked. It may be necessary to track the position of the tibia, femur, patella, a pointer and any instruments at any particular time, or any one or combination of these. If the tracking system is reliably tracking all of the entities required for the current stage, then the status indicator 662 is coloured green. However, if tracking of a one of the entities is prevented, e.g. because there is an interruption of the line of sight between the cameras and the marker on the tracked entity, then the colour of tracking status indicator 662 can change. In one embodiment, if a one of the entities is no longer being reliably tracked then the tracking status indicator can change colour to orange or amber to alert the surgeon. If a second entity is also not being tracked reliably then the tracking status indicator 662 can further change colour to red to visually warn the surgeon of the situation.

In an alternate embodiment, the tracking indicator 662 can change its visual appearance to also identify the entity which is no longer being successfully tracked. For example if an entity is not being reliably tracked then the tracking indicator 662 can change colour to red and the initial of the entity which is not successfully being tracked may be displayed within the tracking status indicator, e.g. "F" for femoral marker, "T" for tibial marker, "P" for a patella and "I" for any instrument or instruments. If more than one of the entities being tracked is not successfully being tracked, then either the initials of all of the entities not being successfully tracked are displayed in the tracking status indicator 662 or alternatively the initials of the entities not reliably being tracked can be displayed sequentially in the tracking status indicator 662.

In a further embodiment, if more than one entity is not being reliably tracked, then a tracking status indicator is displayed in header 402 including the initial of the further entity not being reliably tracked for each entity not being reliably tracked. Hence, the surgeon is provided with a visual guide to the reliability of tracking and an indication of which of the multiple tracked entities is not reliably being tracked so that the surgeon can take action to correct the situation.

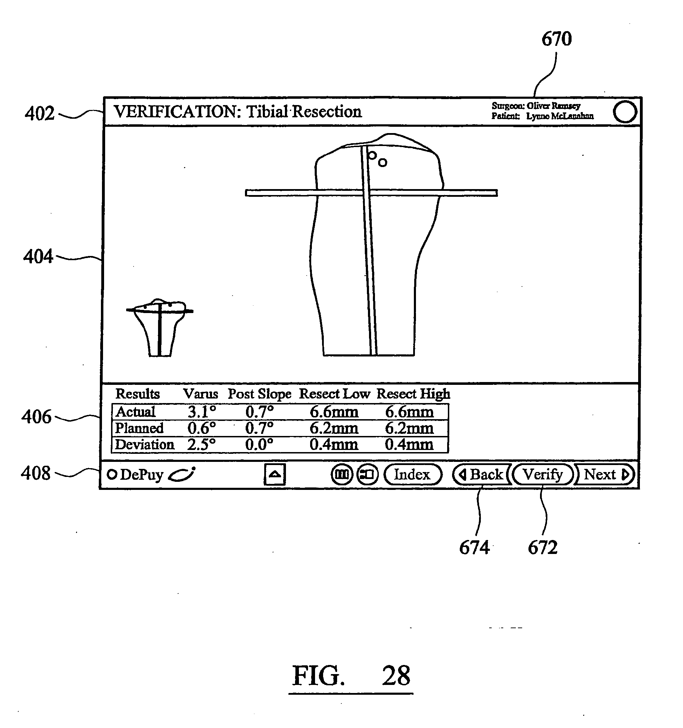

At step 310, navigated positioning of the tibial jig can be carried out until the surgeon is happy that the position of the jig sufficiently closely corresponds to the planned position and/or to the position the surgeon selects based on their experience and judgement. When it is determined that navigation of the current entity has been completed at step 310, then process proceeds to step 314 at which the surgeon can verify the navigated position of the entity. Figure 28 shows a screen shot of a verification screen 670 which can be displayed at step 314. The screen shows an image of the model together with an indication of the planned and navigated positions in a one up display mode in working portion 404. Information relating to the planned and actual position together with deviations there from is displayed in information portion 406 so that the surgeon can check the navigated position of the tibial cutting block before carrying out the tibial cut. If the surgeon is happy with the navigated position of the cutting block then the surgeon can verify the position by pressing verify button 672. Alternatively, if the surgeon wants to reposition the cutting block then the surgeon can return to the navigation screens by using back navigation button 674.

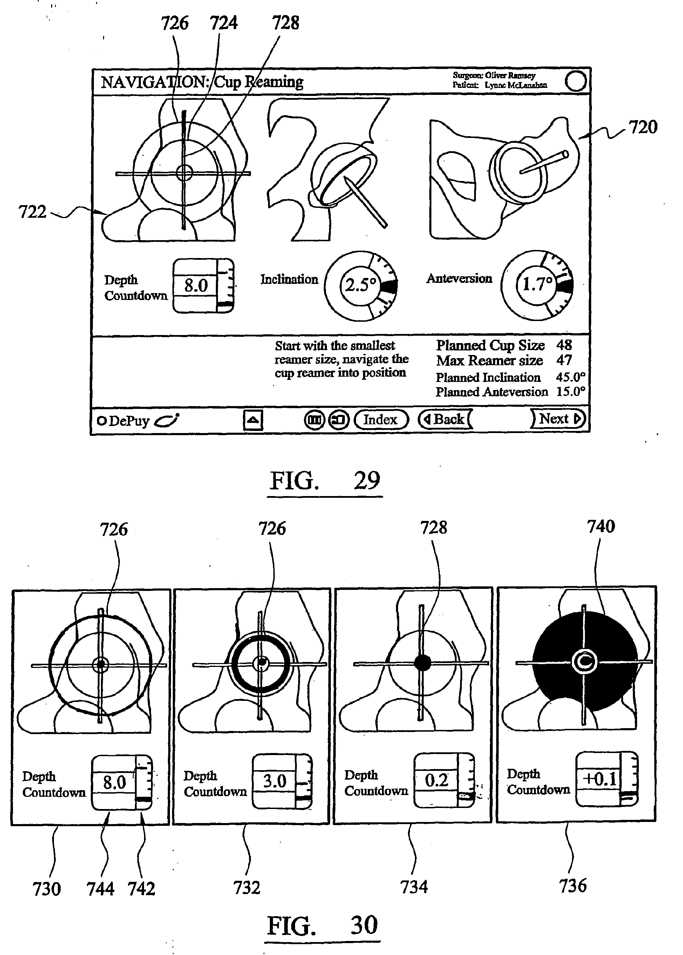

Figure 29 shows a screen shot 720 of a navigation screen of the user interface in a three-up mode. Navigated reaming of an acetabulum is illustrated by the embodiment shown. The navigation screen includes a depth gauge or aiming function which provides a graphical indication of whether the navigated position of the instrument or implant corresponds with the planned position. The depth gauge is in the form of a reticle comprising a pair of concentric circles 726, 728 and a cross hairs 724 as shown in the first image portion 722 in Figure 29. The depth gauge uses dynamic colour and size changes to provide real -time feedback about the depth and orientation of the instrument, which in the described embodiment is a reamer for an acetabular cup implant.

The inner circle's orientation with respect fo the outer circle 726 reflects the inclination and anteversion of the instrument. When the instrument is outside of tolerance of the planned position, the outer circle is coloured red. Figure 30, shows close ups of the targeting feature shown in Figure 29 with the instrument in different positions. As illustrated by close up 730, when the reamer is on target, the outer circle 726 is coloured green. As the reamer moves deeper into the acetabulum, the outer circle 726 gets smaller as shown by close up 732. When the reamer reaches the planned depth, the outer circle disappears and the centre circle 728 is coloured green as shown in close up 734. When the reamer moves beyond the planned depth, the interior of the reticle is coloured red 740 as illustrated in close up 736. A meter 742 is also provided giving an indication of the position of the instrument relative to the planned position and a numerical indication 744 of the depth relative to the planned position.

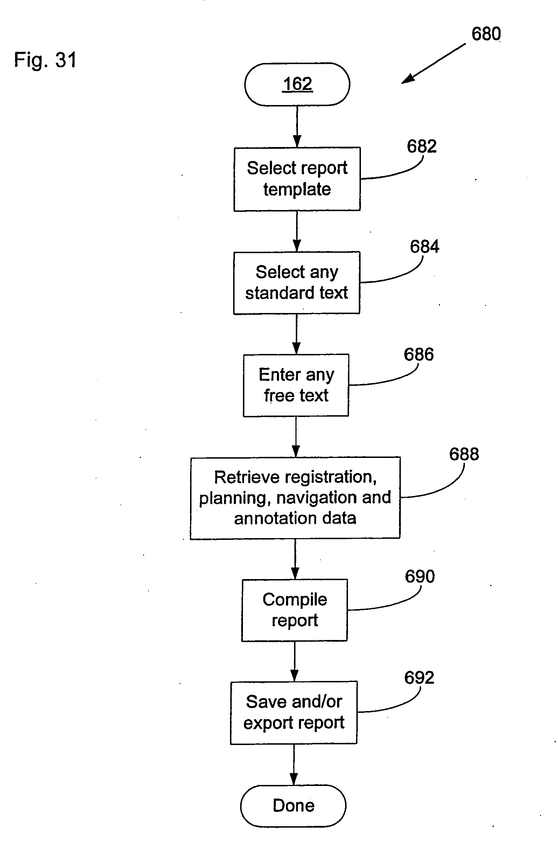

Depending on the workflow, various combinations of steps of planning, registration and navigation may be carried out until the surgical procedure defined by the current workflow has been completed. After the surgical procedure has been completed then at step 162, various reporting activities can optionally be carried out via the user interface. Figure 31 shows a process flowchart illustrating a reporting process 680 corresponding generally to step 162 of Figure 3. Reporting process 680 allows the surgeon to prepare some or all of a report on the procedure using information or data entered into or provided by the user interface while carrying out the procedure. At step 682, the surgeon can select a template for a standard report or for a customised report as previously set up. Alternatively, based on the workflow used, a report template can be generated. At step 684, standard text to be used in the report can be selected by the surgeon. The standard text may include spaces in which information or data specific to the procedure carried out may be automatically inserted. For example the type of implants used, the planned positions, the actual positions and any other data available from the user interface. At step 686, the surgeon can select to enter free text, either via a soft keyboard or hardware keyboard. Alternatively, the surgeon may dictate wording to be included in the report which is converted into text by speech recognition software which processes the audio signal to generate corresponding text.

At step 688, various types of information and data resulting from the workflow is retrieved, such as registration, planning and navigation data. Further, any annotation or comments entered by the surgeon can also selectively be retrieved for inclusion in the report. Then at step 690, the report is compiled using the report template and the text selected and entered by the surgeon. Compiling the report includes integrating the retrieved data into the wording of the report. Then at step 692 the report can be saved locally to the CAS system and/or the report can be exported over a network to a network storage device which provides a central reporting repository. The report can then be accessed by the surgeon over the network for further editing and/or completion. Also, information or data from the report can be extracted from the report to generate reports for use by other medical professionals. For example a nursing report may be prepared which includes surgeon's comments on the operation and/or surgeon's suggestions for the subsequent treatment of the patient to guide the nurse. As a further example, a physiotherapist's report can be generated from the master report which includes details of the implants and reconstruction of the joint which can be used by the physiotherapist to plan suitable physiotherapy and rehabilitation of the patient.

Generally, embodiments of the present invention employ various processes involving data stored in, processed by or transferred through one or more computers or computer systems. Embodiments of the present invention also relate to an apparatus for performing these operations. This apparatus may be specially constructed for the required purposes, or it may be a general-purpose computer selectively activated or reconfigured by a computer program and/or data structure stored in the computer. The processes presented herein are not inherently related to any particular computer or other apparatus. In particular, various general-purpose machines may be used with programs written in accordance with the teachings herein, or it may be more convenient to construct a more specialized apparatus to perform the required method steps. A particular structure for a variety of these machines will appear from the description given below.

In addition, embodiments of the present invention relate to computer readable media or computer program products that include program instructions and/or data (including data structures) for performing various computer-implemented operations. Examples of computer-readable media include, but are not limited to, magnetic media such as hard disks, floppy disks, and magnetic tape; optical media such as CD-ROM disks; magnetooptical media; semiconductor memory devices, and hardware devices that are specially configured to store and perform program instructions, such as read-only memory devices (ROM) and random access memory (RAM). The data and program instructions of this invention may also be embodied on a carrier wave or other transport medium. Examples of program instructions include both machine code, such as produced by a compiler, and files containing higher level code that may be executed by the computer using an interpreter.

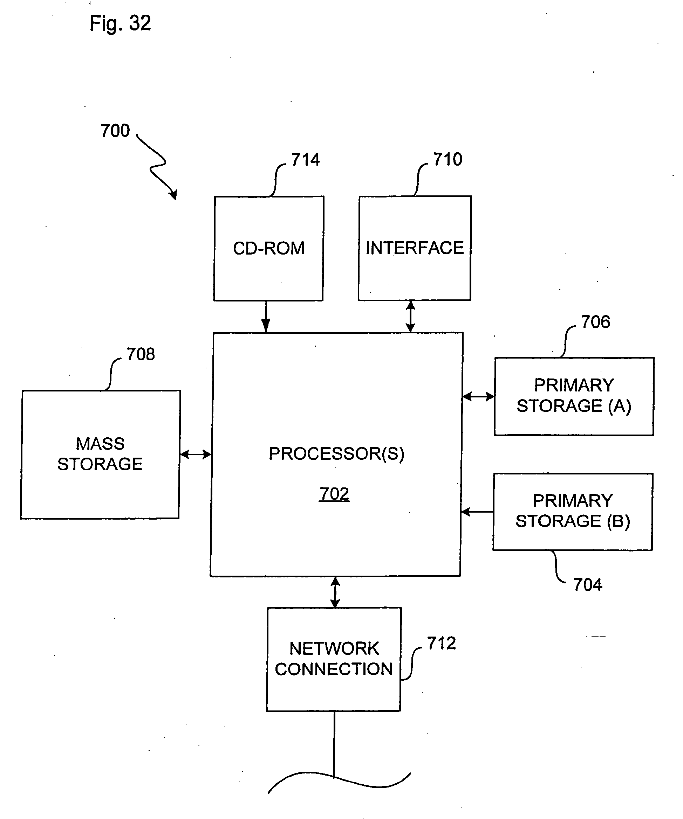

Figure 32 illustrates a typical computer system that, when appropriately configured or designed, can provide the user interface of the invention and serve as a part of a computer assisted surgery system of this invention. The computer system 700 includes any number of processors 702 (also referred to as central processing units, or CPUs) that are coupled to storage devices including primary storage 706 (typically a random access memory, or RAM), primary storage 704 (typically a read only memory, or ROM). CPU 702 may be of various types including microcontrollers and microprocessors such as programmable devices (e.g., CPLDs and FPGAs) and unprogrammable devices such as gate array ASICs or general purpose microprocessors. As is well known in the art, primary storage 704 acts to transfer data and instructions uni-directionally to the CPU and primary storage 706 is used typically to transfer data and instructions in a bi-directional manner. Both of these primary storage devices may include any suitable computer-readable media such as those described above. A mass storage device 708 is also coupled bi-directionally to CPU 702 and provides additional data storage capacity and may include any of the computer-readable media described above. Mass storage device 708 may be used to store programs, data and the like and is typically a secondary storage medium such as a hard disk. It will be appreciated that the information retained within the mass storage device 708, may, in appropriate cases, be incorporated in standard fashion as part of primary storage 706 as virtual memory. A specific mass storage device such as a CD-ROM 714 may also pass data uni-directionally to the CPU.

CPU 702 is also coupled to an interface 710 that connects to one or more input/output devices such as such as video monitors, track balls, mice, keyboards, microphones, touch-sensitive displays, transducer card readers, magnetic or paper tape readers, tablets, styluses, voice or handwriting recognizers, or other well-known input devices such as, of course, other computers. Finally, CPU 702 optionally may be coupled to an external device such as a database or a computer or telecommunications network using an external connection as shown generally at 712. With such a connection, it is contemplated that the CPU might receive information from the network, or might output information to the network in the course of performing the method steps described herein.

Although the above has generally described the present invention according to specific processes and apparatus, the present invention has a much broader range of applicability. In particular, aspects of the present invention is not limited to any particular kind of orthopaedic procedure and can be applied to virtually any kind of surgical or orthopaedic procedure in which planning and an implant or implants are involved. One of ordinary skill in the art would recognize other variants, modifications and alternatives in light of the foregoing discussion.

It will also be appreciated that the invention is not limited to the specific combinations of structural features, data processing operations, data structures or sequences of method steps described and that, unless the context requires otherwise, the specific embodiments described above can be altered, varied and modified. For example different combinations of features of the user interface can be used and features described with reference to one embodiment can be combined with other features described with reference to other embodiments. Similarly the sequence of the data processing operations can be altered and various actions can be combined into a single method step and some methods steps can be carried out as a plurality of individual steps. Further some data processing steps may be carried out in parallel. Also some of the features of the user interface and computer assisted surgery system are schematically illustrated separately, or as comprising particular combinations of features, for the sake of clarity of explanation only. Various of the features and structures can be combined or integrated together or different features assigned to other structures.

## Claims

1. A graphical user interface (400) of a computer assisted surgery system (102) for use by a surgeon during steps of a workflow for a surgical procedure being carried out on a patient, the user interface comprising:
a working portion (404) extending across the user interface in which a plurality of images of different views of a body part of the patient are displayed during the steps of the workflow, and in which the plurality of images are separate from other elements of the user interface;
an information portion (406) positioned below the working portion and extending across the user interface in which information relating to a current step of the workflow illustrated by the images currently displayed in the working portion is displayed;
a control element (436) which can be activated to import a surgeon profile of the surgeon, wherein the surgeon profile includes data relating to how the surgical workflow is customised, so as to allow the computer assisted surgery system to be customised according to the preferences of the surgeon; and
a control bar (408) positioned below the information portion and extending across the user interface and including a plurality of control elements which can be operated by a user of the computer assisted surgery system to operate the computer assisted surgery system.

2. A graphical user interface (400) as claimed in claim 1, in which all control elements (410) which cause the graphical user interface to transition between screens are located in the same region of the control bar (408).

3. A graphical user interface (400) as claimed in claim 1, in which the control bar (408) includes a control element (420) for activating a tool bar (422) providing a plurality of control elements which can be used to control tools provided by the graphical user interface.

4. A graphical user interface (400) as claimed in claim 1, in which the working portion (404) has a first display mode in which the images of different views of the body part (562, 564, 566) are different sizes and a second mode in which the images of different views of the body part (576, 578, 582) are the same size and in which the control bar includes a control element (418, 416) for switching between the first and second display modes.

5. A graphical user interface (400) as claimed in claim 1, in which the working portion (404) has a display mode in which the images of different views of the body part are different sizes and include a main image (562) and at least a first secondary image (564, 566) smaller than the main image, wherein the user can swop the views being displayed as the main image and the secondary image by selecting the secondary image (56, 566).

6. A graphical user interface (400) as claimed in claim 1, in which the control bar (408) includes a control element (526) which can be operated to overlay a list of actions (532) and in which the list of actions indicates which actions have been carried out and which actions are to be carried out.

7. A graphical user interface (400) as claimed in claim 6, in which an item can be selected from the list of actions (532) to navigate the graphical user interface to a screen associated with the selected item.

8. A graphical user interface (400) as claimed in claim 1, in which the control bar includes a control element which can be activated to allow an annotation associated with a current step to be added.

9. A graphical user interface (400) as claimed in claim 1, wherein the annotation is input verbally and converted into text via a speech to text converter.

10. A graphical user interface (400) as claimed in claim 1, and including a control element which can be activated to cause the user interface to output guidance relating to a current step.

11. A graphical user interface (400) as claimed in claim 1, and including a user selection screen (430) via which a current user of the computer assisted surgery system can be identified.

12. A graphical user interface (400) as claimed in claim 1, wherein the user interface is configured to import the surgeon profile from a local database (139) or from a remote database over a network.

13. A graphical user interface (400) as claimed in claim 1, and including a control element (440) which can be activated to export a surgeon profile to a storage medium so that the surgeon profile can be used on a different computer assisted surgery system.

14. A graphical user interface (400) as claimed in claim 1, and including a control element (454) which can be activated to select a standard workflow for carrying out the surgical procedure.

15. A graphical user interface (400) as claimed in claim 1, and including a control element (452) which can be activated to select a customised workflow for carrying out the surgical procedure.

16. A graphical user interface (400) as claimed in claim 1, and including a screen (460) including a plurality of control elements operable to set a plurality of settings used by a workflow for carrying out the surgical procedure.

17. A graphical user interface (400) as claimed in claim 1, and including a screen (490) including a plurality of control elements operable to define a strategy to be used during a workflow for carrying out the surgical procedure.

18. A graphical user interface (400) as claimed in claim 1, and including a screen (500) including a plurality of control elements operable to determine the display mode of the working portion (404) of the graphical user interface at different steps in the workflow for carrying out the surgical procedure.

19. A graphical user interface (400) as claimed in claim 1, and including a registration screen (520) allowing a user to recapture a registration point on a body part by selecting a graphical indication (522, 524) of the point displayed in the working portion (404) of the user interface.

20. A graphical user interface (400) as claimed in claim 1, and including a registration screen (540) which includes a progress metre (542) which provides a linear indication of the proportion of completion of the collection of points on the surface of the body part.

21. A graphical user interface (400) as claimed in claim 1, wherein a first image (552) in the working portion is an image of the surface of a body part and the position of a point on the surface of the body part is indicated by a cross hair target (554) and wherein a second image in the working portion is a cross section through the body part along the line of a one of the cross hairs.

22. A graphical user interface (400) as claimed in claim 21, wherein the cross hairs (554) are displayed in different colours and the cross section is colour coded with the respective cross hair.

23. A graphical user interface (400) as claimed in claim 1, wherein at least one screen (560, 580) includes a widget (568, 570, 572) for setting a value of a planning property, wherein the widget includes a first control element for incrementing the value, a second control element for decrementing the value and a display of the current value.

24. A graphical user interface (400) as claimed in claim 23, wherein the widget (568, 570, 572) further comprises text describing the planning property.

25. A graphical user interface (400) as claimed in claim 4, wherein in the first display mode (560), a plurality of control elements (568, 570, 572) are displayed to one side of the displayed images and in the second display mode (580), a plurality of control elements (568, 570, 572) are displayed below the displayed images.

26. A graphical user interface (400) as claimed in claim 1, wherein at least one control element (574) is displayed in the information portion.

27. A graphical user interface (400) as claimed in claim 1, wherein the user interface includes a navigation screen (640, 650) including at least one widget (656, 658, 660) comparing the determined position of an entity with the planned position of the entity, the or each widget graphically displaying a target value for a planning property, a current value for the planning property and the value of the target value.

28. A graphical user interface (400) as claimed in claim 27, wherein the or each widget (656, 658, 660) further includes a graphical indication of a range of acceptable target values.

29. A graphical user interface (400) as claimed in claim 28, wherein at least some of the or each widget (656, 658, 660) changes colour when the current value of the planning property is within the range of acceptable target values.

30. A graphical user interface (400) as claimed in claim 28 or 29, in which the range of acceptable target values is defined by user settings.

31. A graphical user interface (400) as claimed in claim 1, and including a planning screen (630) which includes a widget (636) for adjusting the planned position of an implant over a plane, the widget including four control elements in a cruciform arrangement.

32. A graphical user interface (400) as claimed in claim 1, and including a navigation screen which includes a control element representing an instrument to be used during the current step of the surgical procedure and wherein the control element can be actuated to display information relating to the instrument.

33. A graphical user interface (400) as claimed in claim 1, wherein the surgical procedure (150) comprises a plurality of general steps (156, 158, 160, 162) and each general step comprises a plurality of sub steps and each screen of the user interface associated with a sub step indicates the proportion of completion of the general step.

34. A graphical user interface (400) as claimed in claim 1, wherein the user interface includes a visual tracking status indicator (434) which indicates whether entities are reliably being tracked by a tracking part of the computer assisted surgery system.

35. A graphical user interface (400) as claimed in claim 34, wherein the tracking status indicator (434) changes colour when at least one entity is not being tracked reliably.

36. A graphical user interface (400) as claimed in claim 35, wherein the tracking status indicator (434) also displays a visual indication of the identity of the entity which is not being tracked reliably.

37. A graphical user interface (400) as claimed in claim 36, wherein the visual indication (434) is a character corresponding to the name of the entity which is not being tracked reliably.

38. A graphical user interface (400) as claimed in claim 1, and including at least one screen including a control element which can be operated to generate a report on the surgical procedure including at least some data entered into the computer assisted surgery system via the user interface.

39. A graphical user interface (400) as claimed in claim 38, and further including a control element which can be operated to export the report from the computer assisted surgery system to a storage device over a network.

40. A graphical user interface (400) as claimed in claim 38 or 39, wherein the report includes information selected from the group comprising: workflow data; implant data; planned position data; actual navigated position data; blood loss data; anaesthesia data; follow up treatment data; physiotherapy data; comment or annotation data entered by the surgeon; and any combination thereof.

41. A computer assisted surgery system (102) for use by a surgeon during steps of a workflow for a surgical procedure being carried out on a patient, the computer assisted surgery system (102) including at least one data processing device (702), a display device (112) and at least one memory (704) holding computer program instructions which can configure the data processing device to display a graphical user interface (400) on the display device, the graphical user interface comprising:
a working portion (404) extending across the user interface in which a plurality of images of different views of a body part of the patient are displayed during the steps of the workflow, and in which the plurality of images are separate from other elements of the user interface;
an information portion (406) positioned below the working portion and extending across the user interface in which information relating to a current step of the workflow illustrated by the images currently displayed in the working portion is displayed;
a control element (436) which can be activated to import a surgeon profile of the surgeon, wherein the surgeon profile includes data relating to how the surgical workflow is customised, so as to allow the computer assisted surgery system to be customised according to the preferences of the surgeon; and
a control bar (408) positioned below the information portion and extending across the user interface and including a plurality of control elements which can be operated by the surgeon to operate the computer assisted surgery system.

42. A method for providing a graphical user interface (400) of a computer assisted surgery system (102) for use by a surgeon during steps of a workflow for a surgical procedure being carried out on a patient, the method comprising:
displaying a plurality of images of different views of a body part of the patient during the steps of the workflow in a working portion (404) of the user interface extending across the user interface, and in which the plurality of images are separate from other elements of the user interface;
displaying information relating to a current step of the workflow illustrated by the images currently displayed in the working portion in an information portion (406) of the user interface positioned below the working portion and extending across the user interface;
displaying a plurality of control elements in a control bar (408) of the user interface positioned below the information portion and extending across the user interface;
importing (174, 190) a surgeon profile of the surgeon, wherein the surgeon profile includes data relating to how the surgical workflow is customised, so as to allow the computer assisted surgery system to be customised according to the preferences of the surgeon; and
operating (156, 158, 160, 162) the computer assisted surgery system in response to operation of control elements displayed in the user interface by the surgeon according to the customised workflow.

43. A computer program product comprising a computer readable medium bearing computer program code providing instructions which can be carried out by a data processing device to provide a graphical user (400) interface of a computer assisted surgery system (102) for use by a surgeon during steps of a workflow for a surgical procedure being carried out on a patient, the instructions including instructions to:
display a plurality of images of different views of a body part of the patient during the steps of the workflow in a working portion (404) of the user interface and extending across the user interface, and in which the plurality of images are separate from other elements of the user interface;
display information relating to a current step of the workflow illustrated by the images currently displayed in the working portion in an information portion (406) of the user interface positioned below the working portion and extending across the user interface;
display a plurality of control elements in a control bar (408) of the user interface positioned below the information portion and extending across the user interface;
import (174, 190) a surgeon profile of the surgeon, wherein the surgeon profile includes data relating to how the surgical workflow is customised, so as to allow the computer assisted surgery system to be customised according to the preferences of the surgeon; and
operate (156, 158, 160, 162) the computer assisted surgery system in response to operation of control elements displayed in the user interface by the surgeon according to the customised workflow.

## Patentansprüche

1. Grafische Benutzerschnittstelle (400) eines computerunterstützten chirurgischen Systems (102) zur Verwendung von einem Chirurgen während der Schritte eines Arbeitsablaufs für eine chirurgische Prozedur, die bei einem Patienten durchgeführt wird, wobei die Benutzerschnittstelle aufweist:
einen Arbeitsbereich (404), der sich über die Benutzerschnittstelle erstreckt, in dem eine Vielzahl von Bildern unterschiedlicher Ansichten eines Körperteiles des Patienten während der Schritte des Arbeitsablaufs angezeigt werden und in dem die Vielzahl der Bilder von anderen Elementen der Benutzerschnittstelle getrennt sind;
einen Informationsbereich (406), der unterhalb des Arbeitsbereiches angeordnet ist und sich über die Benutzerschnittstelle erstreckt, wobei Information, die sich auf einen aktuellen Schritt des Arbeitsablaufes bezieht, der durch die Bilder veranschaulicht wird, die aktuell in dem Arbeitsbereich angezeigt werden, angezeigt wird;
ein Steuerelement (436), das aktiviert werden kann, um ein Chirurgenprofil des Chirurgen zu importieren, wobei das Chirurgenprofil Daten umfasst, die sich darauf beziehen, wie der chirurgische Arbeitsablauf persönlich angepasst wird, um es so dem computerunterstützten chirurgischen System zu ermöglichen, entsprechend den Präferenzen des Chirurgen angepasst zu werden; und
eine Steuerleiste (408), die unterhalb des Informationsbereiches angeordnet ist und sich über die Benutzerschnittstelle erstreckt und eine Vielzahl von Steuerelementen umfasst, die von dem Benutzer des computerunterstützten chirurgischen Systems betätigt werden können, um das computerunterstützte chirurgische System zu betreiben.

2. Grafische Benutzerschnittstelle (400) nach Anspruch 1, bei dem sich alle Steuerelemente (410), die bewirken, dass die grafische Benutzerschnittstelle einen Übergang zwischen Bildschirmen vornimmt, sich in demselben Bereich der Steuerleiste (408) befinden.

3. Grafische Benutzerschnittstelle (400) nach Anspruch 1, bei der die Steuerleiste (408) ein Steuerelement (420) zum Aktivieren einer Werkzeugleiste (422) umfasst, die eine Vielzahl von Steuerelementen bereitstellt, welche verwendet werden können, um Werkzeuge zu steuern, die von der grafischen Benutzerschnittstelle bereitgestellt werden.

4. Grafische Benutzerschnittstelle (400) nach Anspruch 1, bei der der Arbeitsbereich (404) einen ersten Anzeigemodus, in dem die Bilder unterschiedlicher Ansichten des Körperteils (562, 564, 566) unterschiedliche Größen haben und einen zweiten Modus, in dem die Bilder der unterschiedlichen Ansichten des Körperteils (576, 578, 582) die gleiche Größe haben, hat, und bei der die Steuerleiste ein Steuerelement (418, 416) zum Umschalten zwischen dem ersten und dem zweiten Anzeigemodus umfasst.

5. Grafische Benutzerschnittstelle (400) nach Anspruch 1, bei dem der Arbeitsbereich (404) einen Anzeigemodus hat, in dem die Bilder unterschiedlicher Ansichten des Körperteils unterschiedliche Größen haben und ein Hauptbild (562) und wenigstens ein erstes nachrangiges Bild (564, 566), das kleiner ist als das Hauptbild, umfassen, wobei der Benutzer zwischen den Ansichten tauschen kann, die als Hauptbild und nachrangiges Bild angezeigt werden, indem das nachrangige Bild (56, 566) ausgewählt wird.

6. Grafische Benutzerschnittstelle (400) nach Anspruch 1, bei der die Steuerleiste (408) ein Steuerelement (526) umfasst, das betätigt werden kann, um eine Liste von Tätigkeiten (532) aufzulegen, und bei der die Liste der Tätigkeiten angibt, welche Tätigkeiten ausgeführt worden sind und welche Tätigkeiten ausgeführt werden sollen.

7. Grafische Benutzerschnittstelle (400) nach Anspruch 6, bei der ein Punkt aus der Liste der Tätigkeiten (532) ausgewählt werden kann, um die grafische Benutzerschnittstelle zu einem Bildschirm zu führen, der dem ausgewählten Punkt zugeordnet ist.

8. Grafische Benutzerschnittstelle (400) nach Anspruch 1, bei der die Steuerleiste ein Steuerelement umfasst, das aktiviert werden kann, um zu ermöglichen, dass eine Kommentierung, die mit einem aktuellen Schritt verknüpft ist, hinzugefügt wird.

9. Grafische Benutzerschnittstelle (400) nach Anspruch 1, bei der die Kommentierung durch Sprache eingegeben wird und durch einen Sprachumwandler in einen Text umgewandelt wird.

10. Grafische Benutzerschnittstelle (400) nach Anspruch 1 und ein Steuerelement umfassend, das aktiviert werden kann, um zu bewirken, dass die Benutzerschnittstelle eine Führung ausgibt, die in Bezug zu einem aktuellen Schritt steht.

11. Grafische Benutzerschnittstelle (400) nach Anspruch 1 und einen Benutzerauswahlbildschirm (430) umfassend, über den ein aktueller Benutzer des computerunterstützten chirurgischen Systems identifiziert werden kann.

12. Grafische Benutzerschnittstelle (400) nach Anspruch 1, bei der die Benutzerschnittstelle so ausgelegt ist, dass sie das Profil des Chirurgen aus einer lokalen Datenbank (139) oder von einer entfernt befindlichen Datenbank über ein Netzwerk importiert.

13. Grafische Benutzerschnittstelle (400) nach Anspruch 1 und ein Steuerelement (440) umfassend, das aktiviert werden kann, um ein Profil eines Chirurgen zu einem Speichermedium zu exportieren, so dass das Profil des Chirurgen auf einem unterschiedlichen computerunterstützten chirurgischen System verwendet werden kann.

14. Grafische Benutzerschnittstelle (400) nach Anspruch 1 und ein Steuerelement (454) umfassend, das aktiviert werden kann, um einen standardmäßigen Arbeitsablauf zum Durchführen der chirurgischen Prozedur auszuwählen.

15. Grafische Benutzerschnittstelle (400) nach Anspruch 1 und ein Steuerelement (452) umfassend, das aktiviert werden kann, um einen persönlich angepassten Arbeitsablauf zum Durchführen der chirurgischen Prozedur auszuwählen.

16. Grafische Benutzerschnittstelle (400) nach Anspruch 1 und einen Bildschirm (460) umfassend, der eine Vielzahl von Steuerelementen umfasst, die so betreibbar sind, dass eine Vielzahl von Einstellungen eingerichtet werden kann, die von einem Arbeitsablauf zum Durchführen der chirurgischen Prozedur verwendet werden.

17. Grafische Benutzerschnittstelle (400) nach Anspruch 1 und einen Bildschirm (490) umfassend, der eine Vielzahl von Steuerelementen umfasst, die so betreibbar sind, dass sie eine Strategie definieren, die während eines Arbeitsablaufes zum Durchführen der chirurgischen Prozedur verwendet werden soll.

18. Grafische Benutzerschnittstelle (400) nach Anspruch 1 und einen Bildschirm (500) umfassend, der eine Vielzahl von Steuerelementen umfasst, die so betreibbar sind, dass sie den Anzeigemodus des Arbeitsbereiches (404) der grafischen Benutzerschnittstelle bei unterschiedlichen Schritten in dem Arbeitsablauf zum Durchführen der chirurgischen Prozedur festlegen.

19. Grafische Benutzerschnittstelle (400) nach Anspruch 1 und einen Registrierbildschirm (520) umfassend, der es einem Benutzer erlaubt, einen Ausrichtepunkt auf einem Körperteil wieder einzufangen, indem eine grafische Angabe (522, 524) des Punktes ausgewählt wird, der in dem Arbeitsbereich (404) der Benutzerschnittstelle angezeigt wird.

20. Grafische Benutzerschnittstelle (400) nach Anspruch 1 und einen Registrierbildschirm (540) umfassend, der einen Fortschrittsmesser (542) umfasst, der eine lineare Angabe des Anteils der Vervollständigung der Punktesammlung auf der Oberfläche des Körperteiles liefert.

21. Grafische Benutzerschnittstelle (400) nach Anspruch 1, bei der ein erstes Bild (552) in dem Arbeitsbereich ein Bild der Oberfläche eines Körperteiles ist und die Position eines Punktes auf der Oberfläche des Körperteiles durch ein Fadenkreuz (554) angegeben wird, und bei der ein zweites Bild in dem Arbeitsbereich ein Querschnitt durch den Körperteil entlang der Linie eines der Fadenkreuze ist.

22. Grafische Benutzerschnittstelle (400) nach Anspruch 21, bei der die Fadenkreuze (554) in unterschiedlichen Farben angezeigt werden und der Querschnitt mit dem jeweiligen Fadenkreuz farbcodiert ist.

23. Grafische Benutzerschnittstelle (400) nach Anspruch 1, bei der wenigstens ein Bildschirm (560, 580) ein Grafikobjekt (568, 570, 572) zum Einstellen eines Wertes für eine geplante Eigenschaft umfasst, wobei das Grafikobjekt ein erstes Steuerelement zum Inkrementieren des Wertes, ein zweites Steuerelement zum Dekrementieren des Wertes und eine Anzeige des aktuellen Wertes umfasst.

24. Grafische Benutzerschnittstelle (400) nach Anspruch 23, bei der das Grafikobjekt (568, 570, 572) weiter Text aufweist, der die geplante Eigenschaft beschreibt.

25. Grafische Benutzerschnittstelle (400) nach Anspruch 4, bei der in dem ersten Anzeigemodus (560) eine Vielzahl von Steuerelementen (568, 570, 572) auf einer Seite der angezeigten Bilder angezeigt wird und in dem zweiten Anzeigemodus (580) eine Vielzahl von Steuerelementen (568, 570, 572) unterhalb der angezeigten Bilder angezeigt werden.

26. Grafische Benutzerschnittstelle (400) nach Anspruch 1, bei der wenigstens ein Steuerelement (574) in dem Informationsbereich angezeigt wird.

27. Grafische Benutzerschnittstelle (400) nach Anspruch 1, bei der die Benutzerschnittstelle einen Navigationsbildschirm (640, 650) umfasst, der wenigstens ein Grafikobjekt (656, 658, 660) umfasst, welches die festgestellte Position einer Einheit mit der geplanten Position der Einheit vergleicht, wobei das oder jedes Grafikobjekt grafisch einen Zielwert für eine geplante Eigenschaft, einen aktuellen Wert für die geplante Eigenschaft und den Wert des Zielwertes anzeigt.

28. Grafische Benutzerschnittstelle (400) nach Anspruch 27, bei der das oder jedes Grafikobjekt (656, 658, 660) weiter eine grafische Angabe eines Bereiches akzeptabler Zielwerte umfasst.

29. Grafische Benutzerschnittstelle (400) nach Anspruch 28, bei der wenigstens einige der oder jedes Grafikobjekt (656, 658, 660) seine Farbe ändert, wenn der aktuelle Wert der geplanten Eigenschaften innerhalb des Bereiches der akzeptablen Zielwerte liegt.

30. Grafische Benutzerschnittstelle (400) nach Anspruch 28 oder 29, bei der der Bereich akzeptabler Zielwerte durch Benutzereinstellungen definiert ist.

31. Grafische Benutzerschnittstelle (400) nach Anspruch 1 und einen Planungsbildschirm (630) umfassend, der ein Grafikobjekt (636) zum Anpassen der geplanten Position eines Implantats über eine Ebene umfasst, wobei das Grafikobjekt vier Steuerelemente in einer kreuzförmigen Anordnung umfasst.

32. Grafische Benutzerschnittstelle (400) nach Anspruch 1 und einen Navigationsbildschirm umfassend, der ein Steuerelement umfasst, welches ein Instrument darstellt, das während des aktuellen Schrittes der chirurgischen Prozedur verwendet werden soll, und bei der das Steuerelement betätigt werden kann, um Information in Bezug auf das Instrument anzuzeigen.

33. Grafische Benutzerschnittstelle (400) nach Anspruch 1, bei der die chirurgische Prozedur (150) eine Vielzahl allgemeiner Schritte (156, 158, 160, 162) aufweist und jeder allgemeine Schritt eine Vielzahl von Teilschritten aufweist und jeder Bildschirm der Benutzerschnittstelle, der einem Teilschritt zugeordnet ist, den Anteil bis zur Vollendung des allgemeinen Schrittes anzeigt.

34. Grafische Benutzerschnittstelle (400) nach Anspruch 1, bei der die Benutzerschnittstelle einen Indikator (434) für das visuelle Verfolgen des Status umfasst, der anzeigt, ob Einheiten zuverlässig von einem Verfolgerteil des computerunterstützten chirurgischen Systems verfolgt werden.

35. Grafische Benutzerschnittstelle (400) nach Anspruch 34, bei der der Indikator (434) für das Verfolgen des Status seine Farbe wechselt, wenn wenigstens eine Einheit nicht zuverlässig verfolgt wird.

36. Grafische Benutzerschnittstelle (400) nach Anspruch 35, bei der der Indikator (434) zum Verfolgen des Status außerdem eine visuelle Anzeige der Identität der Einheit, die nicht zuverlässig verfolgt wird, anzeigt.

37. Grafische Benutzerschnittstelle (400) nach Anspruch 36, bei der die visuelle Angabe (434) ein Zeichen ist, das dem Namen der Einheit entspricht, die nicht zuverlässig verfolgt wird.

38. Grafische Benutzerschnittstelle (400) nach Anspruch 1 und wenigstens einen Bildschirm umfassend, der ein Steuerelement umfasst, das betätigt werden kann, um einen Bericht über die chirurgische Prozedur zu erzeugen, der wenigstens einige Daten umfasst, die über die Benutzerschnittstelle in das computerunterstützte chirurgische System eingegeben worden sind.

39. Grafische Benutzerschnittstelle (400) nach Anspruch 38 und weiter ein Steuerelement umfassend, das betrieben werden kann, um den Bericht aus dem computerunterstützten chirurgischen System über ein Netzwerk an eine Speichervorrichtung zu exportieren.

40. Grafische Benutzerschnittstelle (400) nach Anspruch 38 oder 39, bei der der Bericht Information umfasst, die aus der Gruppe aus: Arbeitsablaufdaten; Implantatdaten; Daten über die geplante Position; Daten über die aktuell navigierte Position; Blutverlustdaten; Anästhesiedaten; Nachfolgebehandlungsdaten; Physiotherapiedaten; Kommentar- oder Anmerkungsdaten, die von dem Chirurgen eingegeben worden sind; und irgendeine Kombination aus diesen ausgewählt sind.

41. Computerunterstütztes chirurgisches System (102) zur Verwendung von einem Chirurgen während der Schritt eines Arbeitsablaufs bei einer chirurgischen Prozedur, die bei einem Patienten ausgeführt wird, wobei das computerunterstützte chirurgische System (102) wenigstens eine Datenverarbeitungseinrichtung (702), eine Anzeigevorrichtung (112) und wenigstens einen Speicher (704) umfasst, der Computerprogrammbefehle hält, die die Datenverarbeitungseinrichtung konfigurieren können, um eine grafische Benutzerschnittstelle (400) auf der Anzeigevorrichtung anzuzeigen, wobei die grafische Benutzerschnittstelle aufweist:
einen Arbeitsbereich (404), der sich über die Benutzerschnittstelle erstreckt, in dem eine Vielzahl von Bildern unterschiedlicher Ansichten eines Körperteiles des Patienten während der Schritte des Arbeitsablaufs angezeigt werden und in dem die Vielzahl der Bilder von anderen Elementen der Benutzerschnittstelle getrennt sind;
einen Informationsbereich (406), der unterhalb des Arbeitsbereiches angeordnet ist und sich über die Benutzerschnittstelle erstreckt, wobei Information, die sich auf einen aktuellen Schritt des Arbeitsablaufes bezieht, durch die Bilder veranschaulicht wird, die aktuell in dem Arbeitsbereich angezeigt werden;
ein Steuerelement (436), das aktiviert werden kann, um ein Chirurgenprofil des Chirurgen zu importieren, wobei das Chirurgenprofil Daten umfasst, die sich darauf beziehen, wie der chirurgische Arbeitsablauf persönlich angepasst wird, um es so dem computerunterstützten chirurgischen System zu ermöglichen, entsprechend den Präferenzen des Chirurgen angepasst zu werden; und
eine Steuerleiste (408), die unterhalb des Informationsbereiches angeordnet ist und sich über die Benutzerschnittstelle erstreckt und eine Vielzahl von Steuerelementen umfasst, die von dem Benutzer des computerunterstützten chirurgischen Systems betätigt werden können, um das computerunterstützte chirurgische System zu betreiben.

42. Verfahren zum Bereitstellen einer grafischen Benutzerschnittstelle (400) eines computerunterstützten chirurgischen Systems (102) zur Verwendung von einem Chirurgen während der Schritte eines Arbeitsablaufes bei einer chirurgischen Prozedur, die bei einem Patienten durchgeführt wird, wobei das Verfahren aufweist:
Anzeigen einer Vielzahl von Bildern unterschiedlicher Ansichten eines Körperteils des Patienten während der Schritte des Arbeitsablaufes in einem Arbeitsbereich (404) der Benutzerschnittstelle, die sich über die Benutzerschnittstelle erstrecken, und wobei die Vielzahl der Bilder von anderen Elementen der Benutzerschnittstelle getrennt ist;
Anzeigen von Information in Bezug auf einen aktuellen Schritt des Arbeitsablaufes, der durch die Bilder veranschaulicht wird, die aktuell in dem Arbeitsbereich in einem Informationsbereich (406) der Benutzerschnittstelle angezeigt werden, der unterhalb des Arbeitsbereiches angeordnet ist und sich über die Benutzerschnittstelle erstreckt;
Anzeigen einer Vielzahl von Steuerelementen in einer Steuerleiste (408) der Benutzerschnittstelle, die unterhalb des Informationsbereiches angeordnet ist und sich über die Benutzerschnittstelle erstreckt;
Importieren (174, 190) eines Chirurgenprofils des Chirurgen, wobei das Chirurgenprofil Daten umfasst, die sich darauf beziehen, wie der chirurgische Arbeitsablauf persönlich angepasst ist, um es so dem computerunterstützten chirurgischen System zu ermöglichen, entsprechend den Präferenzen des Chirurgen angepasst zu werden; und
Betreiben (156, 158, 160, 162) des computerunterstützten chirurgischen Systems ansprechend auf das Betätigen der Steuerelemente, die in der Benutzerschnittstelle angezeigt werden, von dem Chirurgen, entsprechend dem angepassten Arbeitsablauf.

43. Computerprogrammprodukt, das ein von einem Computer lesbares Medium aufweist, welches einen Computerprogrammcode trägt, der Befehle bereitstellt, die von einer Datenverarbeitungseinrichtung ausgeführt werden können, um eine grafische Benutzerschnittstelle (400) eines computerunterstützten chirurgischen Systems (102) zur Verwendung von einem Chirurgen bei Schritten eines Arbeitsablaufes für eine chirurgische Prozedur, die bei einem Patienten durchgeführt werden, zur Verfügung zu stellen, wobei die Befehle solche Befehle enthalten, die:
eine Vielzahl von Bildern unterschiedlicher Ansichten eines Körperteils des Patienten während der Schritte des Arbeitsablaufes in einem Arbeitsbereich (404) der Benutzerschnittstelle, die sich über die Benutzerschnittstelle erstrecken, anzeigen und wobei die Vielzahl der Bilder von anderen Elementen der Benutzerschnittstelle getrennt sind;
Information in Bezug auf einen aktuellen Schritt des Arbeitsablaufes anzeigen, der durch die Bilder veranschaulicht wird, die aktuell in dem Arbeitsbereich in einem Informationsbereich (406) der Benutzerschnittstelle angezeigt werden, der unterhalb des Arbeitsbereiches angeordnet ist und sich über die Benutzerschnittstelle erstreckt;
eine Vielzahl von Steuerelementen in einer Steuerleiste (408) der Benutzerschnittstelle anzeigen, die unterhalb des Informationsbereiches angeordnet ist und sich über die Benutzerschnittstelle erstreckt;
ein Chirurgenprofil des Chirurgen importieren (174, 190), wobei das Chirurgenprofil Daten umfasst, die sich darauf beziehen, wie der chirurgische Arbeitsablauf persönlich angepasst ist, um es so dem computerunterstützten chirurgischen System zu ermöglichen, entsprechend den Präferenzen des Chirurgen angepasst zu werden; und
das computerunterstützte chirurgische System ansprechend auf das Betätigen der Steuerelemente, die in der Benutzerschnittstelle angezeigt werden, betreiben (156, 158, 160, 162), von dem Chirurgen, entsprechend dem angepassten Arbeitsablauf.

## Revendications

1. Interface utilisateur graphique (400) d'un système chirurgical assisté par ordinateur (102) à utiliser par un chirurgien pendant les étapes d'un protocole pour une procédure chirurgicale exécutée sur un patient, l'interface utilisateur comprenant :
un espace de travail (404) qui s'étend en travers de l'interface utilisateur, dans laquelle une pluralité d'images de différentes vues d'une partie de corps du patient sont affichées pendant les étapes du protocole, et dans laquelle la pluralité d'images sont séparées d'autres éléments de l'interface utilisateur ;
un espace d'informations (406) qui est positionnée en dessous de l'espace de travail et qui s'étend en travers de l'interface utilisateur, dans laquelle des informations relatives à une étape en cours du protocole illustrée par les images actuellement affichées dans l'espace de travail sont affichées ;
un élément de commande (436) qui peut être activé pour importer un profil de chirurgien du chirurgien, dans laquelle le profil de chirurgien contient des données relatives à la manière avec laquelle le protocole chirurgical est personnalisé, de manière à permettre au système chirurgical assisté par ordinateur d'être personnalisé sur la base des préférences du chirurgien ; et
une barre de commande (408) qui est positionnée en dessous de l'espace d'informations et qui s'étend en travers de l'interface utilisateur, et comprenant une pluralité d'éléments de commande qui peuvent être actionnés par un utilisateur du système chirurgical assisté par ordinateur pour commander le système chirurgical assisté par ordinateur.

2. Interface utilisateur graphique (400) selon la revendication 1, dans laquelle tous les éléments de commande (410) qui entraînent l'interface utilisateur graphique à effectuer une transition entre des écrans sont situés dans la même région de la barre de commande (408).

3. Interface utilisateur graphique (400) selon la revendication 1, dans laquelle la barre de commande (408) comprend un élément de commande (420) pour activer une barre d'outils (422) fournissant une pluralité d'éléments de commande qui peuvent être utilisés pour commander les outils fournis par l'interface utilisateur graphique.

4. Interface utilisateur graphique (400) selon la revendication 1, dans laquelle l'espace de travail (404) présente un premier mode d'affichage dans lequel les images de différentes vues de la partie de corps (562, 564, 566) sont de tailles différentes, et un deuxième mode dans lequel les images de différentes vues de la partie de corps (576, 578, 582) ont la même taille, et dans laquelle la barre de commande comprend un élément de commande (418, 416) pour effectuer une commutation entre les premier et deuxième modes d'affichage.

5. Interface utilisateur graphique (400) selon la revendication 1, dans laquelle l'espace de travail (404) présente un mode d'affichage dans lequel les images de différentes vues de la partie de corps sont de tailles différentes et comprennent une image principale (562) et au moins une première image secondaire (564, 566) qui est plus petite que l'image principale, dans laquelle l'utilisateur peut interchanger les vues qui sont affichées comme image principale et image secondaire en sélectionnant l'image secondaire (56, 566).

6. Interface utilisateur graphique (400) selon la revendication 1, dans laquelle la barre de commande (408) comprend un élément de commande (526) qui peut être actionné pour superposer une liste d'actions (532), et dans laquelle la liste d'actions indique les actions qui ont été exécutées et les actions qui n'ont pas été exécutées.

7. Interface utilisateur graphique (400) selon la revendication 6, dans laquelle un article peut être sélectionné dans la liste d'actions (532) afin de faire naviguer l'interface utilisateur graphique sur un écran associé à l'article sélectionné.

8. Interface utilisateur graphique (400) selon la revendication 1, dans laquelle la barre de commande comprend un élément de commande qui peut être activé pour permettre d'ajouter une annotation associée à une étape en cours.

9. Interface utilisateur graphique (400) selon la revendication 1, dans laquelle l'annotation est entrée verbalement et est convertie en texte par l'intermédiaire d'une parole adressée à un convertisseur de texte.

10. Interface utilisateur graphique (400) selon la revendication 1, et comprenant un élément de commande qui peut être activé pour entraîner l'interface utilisateur graphique à générer un guidage en relation avec une étape en cours.

11. Interface utilisateur graphique (400) selon la revendication 1, et comprenant un écran de sélection d'utilisateur (430) par l'intermédiaire duquel un utilisateur actuel du système chirurgical assisté par ordinateur peut être identifié.

12. Interface utilisateur graphique (400) selon la revendication 1, dans laquelle l'interface utilisateur est configurée de manière à importer le profil de chirurgien à partir d'une base de données locale (139) ou à partir d'une base de données distante sur un réseau.

13. Interface utilisateur graphique (400) selon la revendication 1, et comprenant un élément de commande (440) qui peut être activé pour exporter un profil de chirurgien vers un support de stockage de telle sorte que le profil de chirurgien puisse être utilisé sur un système chirurgical assisté par ordinateur différent.

14. Interface utilisateur graphique (400) selon la revendication 1, et comprenant un élément de commande (454) qui peut être activé pour sélectionner un protocole standard pour exécuter la procédure chirurgicale.

15. Interface utilisateur graphique (400) selon la revendication 1, et comprenant un élément de commande (452) qui peut être activé pour sélectionner un protocole personnalisé pour exécuter la procédure chirurgicale.

16. Interface utilisateur graphique (400) selon la revendication 1, et comprenant un écran (460) contenant une pluralité d'éléments de commande utilisables pour effectuer une pluralité de réglages utilisés par un protocole pour exécuter la procédure chirurgicale.

17. Interface utilisateur graphique (400) selon la revendication 1, et comprenant un écran (490) contenant une pluralité d'éléments de commande utilisables pour définir une stratégie à utiliser pendant un protocole pour exécuter la procédure chirurgicale.

18. Interface utilisateur graphique (400) selon la revendication 1, et comprenant un écran (500) contenant une pluralité d'éléments de commande utilisables pour déterminer le mode d'affichage de l'espace de travail (404) de l'interface utilisateur graphique à différentes étapes du protocole pour exécuter la procédure chirurgicale.

19. Interface utilisateur graphique (400) selon la revendication 1, et comprenant un écran d'enregistrement (520) qui permet à un utilisateur de re-capturer un point d'enregistrement sur une partie de corps en sélectionnant une indication graphique (522, 524) du point affiché dans l'espace de travail (404) de l'interface utilisateur .

20. Interface utilisateur graphique (400) selon la revendication 1, et comprenant un écran d'enregistrement (540) contenant une jauge de progression (542) qui fournit une indication linaire de la proportion d'accomplissement de la collecte de points sur la surface de la partie de corps.

21. Interface utilisateur graphique (400) selon la revendication 1, dans laquelle une première image (552) dans l'espace de travail est une image de la surface d'une partie de corps, et la position d'un point sur la surface de la partie de corps est indiquée par une cible en réticule (554), et dans laquelle une deuxième image dans l'espace de travail est une section transversale à travers la partie de corps le long de la ligne de l'un des réticules.

22. Interface utilisateur graphique (400) selon la revendication 21, dans laquelle les réticules (554) sont affichés dans des couleurs différentes, et la section transversale présente un codage de couleurs qui correspond au réticule respectif.

23. Interface utilisateur graphique (400) selon la revendication 1, dans laquelle au moins un écran (560, 580) comprend un gadget logiciel (568, 570, 572) pour régler une valeur d'une propriété de planification, dans laquelle le gadget logiciel comprend un premier élément de commande pour incrémenter la valeur, un deuxième élément de commande pour décrémenter la valeur, et un affichage de la valeur actuelle.

24. Interface utilisateur graphique (400) selon la revendication 23, dans laquelle le gadget logiciel (568, 570, 572) comprend en outre un texte décrivant une propriété de planification.

25. Interface utilisateur graphique (400) selon la revendication 4, dans laquelle, dans le premier mode d'affichage (560), une pluralité d'éléments de commande (568, 570, 572) sont affichés sur un côté des images affichées et, dans le deuxième mode d'affichage (580), une pluralité d'éléments de commande (568, 570, 572) sont affichés en dessous des images affichées.

26. Interface utilisateur graphique (400) selon la revendication 1, dans laquelle au moins un élément de commande (574) est affiché dans l'espace d'informations.

27. Interface utilisateur graphique (400) selon la revendication 1, dans laquelle l'interface utilisateur comprend un écran de navigation (640, 650) contenant au moins un gadget logiciel (656, 658, 660) qui compare la position déterminée d'une entité avec la position planifiée de l'entité, le ou chaque gadget logiciel affichant graphiquement une valeur cible pour une propriété de planification, une valeur actuelle pour la propriété de planification et la valeur de la valeur cible.

28. Interface utilisateur graphique (400) selon la revendication 27, dans laquelle le ou chaque gadget logiciel (656, 658, 660) comprend en outre une indication d'une gamme de valeurs cibles acceptables.

29. Interface utilisateur graphique (400) selon la revendication 28, dans laquelle au moins certains du ou de chaque gadget logiciel (656, 658, 660) change de couleur lorsque la valeur actuelle de la propriété de planification se situe à l'intérieur de la gamme de valeurs cibles acceptables.

30. Interface utilisateur graphique (400) selon la revendication 28 ou 29, dans laquelle la gamme de valeurs cibles acceptables est définie par des réglages de l'utilisateur.

31. Interface utilisateur graphique (400) selon la revendication 1, et comprenant un écran de planification (630) qui contient un gadget logiciel (636) pour régler la position planifiée d'un implant sur un plan, le gadget logiciel comprenant quatre éléments de commande en un agencement cruciforme.

32. Interface utilisateur graphique (400) selon la revendication 1, et comprenant un écran de navigation contenant un élément de commande qui représente un instrument à utiliser pendant l'étape en cours de la procédure chirurgicale, et dans laquelle l'élément de commande peut être actionné pour afficher des informations relatives à l'instrument.

33. Interface utilisateur graphique (400) selon la revendication 1, dans laquelle la procédure chirurgicale (150) comprend une pluralité d'étapes générales (156, 158, 160, 162), et chaque étape générale comprend une pluralité d'étapes secondaires, et chaque écran de l'interface utilisateur associé à une étape secondaire indique la proportion d'accomplissement de l'étape générale.

34. Interface utilisateur graphique (400) selon la revendication 1, dans laquelle l'interface utilisateur comprend un indicateur de statut de poursuite visuel (434) qui indique si des entités sont suivies de façon fiable par une partie de poursuite du système chirurgical assisté par ordinateur.

35. Interface utilisateur graphique (400) selon la revendication 34, dans laquelle l'indicateur du statut de poursuite (434) change de couleur lorsqu'au moins une entité n'est pas suivie de façon fiable.

36. Interface utilisateur graphique (400) selon la revendication 35, dans laquelle l'indicateur de statut de poursuite (434) affiche également une indication visuelle de l'identité de l'entité qui n'est pas suivie de façon fiable.

37. Interface utilisateur graphique (400) selon la revendication 36, dans laquelle l'indication visuelle (434) est un caractère qui correspond au nom de l'entité qui n'est pas suivie de façon fiable.

38. Interface utilisateur graphique (400) selon la revendication 1, et comprenant au moins un écran contenant un élément de commande qui peut être actionné pour générer un rapport sur la procédure chirurgicale qui contient au moins plusieurs données entrées dans le système chirurgical assisté par ordinateur par l'intermédiaire de l'interface utilisateur .

39. Interface utilisateur graphique (400) selon la revendication 38, et comprenant en outre un élément de commande qui peut être actionné pour exporter le rapport du système chirurgical assisté par ordinateur vers un dispositif de stockage sur un réseau.

40. Interface utilisateur graphique (400) selon la revendication 38 ou 39, dans laquelle le rapport contient des informations qui sont sélectionnées dans le groupe comprenant : des données de protocole ; des données d'implant ; des données de position planifiée ; des données de position naviguée réelle ; des données de perte de sang ; des données d'anesthésie ; des données de traitement de suivi ; des données de physiothérapie ; des données de commentaires ou d'annotations entrées par le chirurgien ; ainsi que toute combinaison de celles-ci.

41. Système chirurgical assisté par ordinateur (102) à utiliser par un chirurgien pendant les étapes d'un protocole pour une procédure chirurgicale exécutée sur un patient, le système chirurgical assisté par ordinateur (102) comprenant au moins un dispositif de traitement de données (702), un système d'affichage (112) et au moins une mémoire (704) comprenant des instructions de programme logiciel qui peuvent configurer le dispositif de traitement de données pour afficher une interface utilisateur graphique (400) sur le dispositif d'affichage, l'interface utilisateur comprenant :
un espace de travail (404) qui s'étend en travers de l'interface utilisateur, dans lequel une pluralité d'images de différentes vues d'une partie de corps sont affichées pendant les étapes du protocole, et dans lequel la pluralité d'images sont séparées d'autres éléments de l'interface utilisateur ;
un espace d'informations (406) qui est positionnée en dessous de l'espace de travail et qui s'étend en travers de l'interface utilisateur, dans lequel des informations relatives à une étape en cours du protocole illustrée par les images actuellement affichées dans l'espace de travail sont affichées ;
un élément de commande (436) qui peut être activé pour importer un profil de chirurgien du chirurgien, dans lequel le profil de chirurgien contient des données relatives à la manière avec laquelle le protocole chirurgical est personnalisé, de manière à permettre au système chirurgical assisté par ordinateur d'être personnalisé sur la base des préférences du chirurgien ; et
une barre de commande (408) qui est positionnée en dessous de l'espace d'informations et qui s'étend en travers de l'interface utilisateur, et comprenant une pluralité d'éléments de commande qui peuvent être actionnés par le chirurgien pour commander le système chirurgical assisté par ordinateur.

42. Procédé pour fournir une interface utilisateur graphique (400) d'un système chirurgical assisté par ordinateur (102) à utiliser par un chirurgien pendant les étapes d'un protocole pour une procédure chirurgicale exécutée sur un patient, le procédé comprenant les étapes suivantes :
afficher une pluralité d'images de différentes vues d'une partie de corps du patient pendant les étapes du protocole dans un espace de travail (404) de l'interface utilisateur qui s'étend en travers de l'interface utilisateur, et dans laquelle la pluralité d'images sont séparées d'autres éléments de l'interface utilisateur ;
afficher une pluralité d'éléments de commande dans une barre de commande (408) de l'interface utilisateur qui est positionnée en dessous de l'espace d'informations et qui s'étend en travers de l'interface utilisateur ;
afficher des informations relatives à une étape en cours du protocole illustrée par les images actuellement affichées dans l'espace de travail dans un espace d'informations (406) de l'interface utilisateur qui est positionnée en dessous de l'espace de travail et qui s'étend en travers de l'interface utilisateur ;
importer (174, 190) un profil de chirurgien du chirurgien, dans laquelle le profil de chirurgien contient des données relatives à la manière avec laquelle le protocole chirurgical est personnalisé, de manière à permettre au système chirurgical assisté par ordinateur d'être personnalisé sur la base des préférences du chirurgien ; et
actionner (156, 158, 160, 162) le système chirurgical assisté par ordinateur en réponse à l'actionnement d'éléments de commande affichés dans l'interface utilisateur par le chirurgien conformément au protocole personnalisé.

43. Produit de programme d'ordinateur comprenant un support lisible par un ordinateur contenant un code de programme d'ordinateur qui fournit des instructions pouvant être exécutées par un dispositif de traitement de données pour former une interface utilisateur graphique (400) d'un système chirurgical assisté par ordinateur (102) à utiliser par un chirurgien pendant les étapes d'un protocole pour une procédure chirurgicale exécutée sur un patient, les instructions comprenant des instructions pour :
afficher une pluralité d'images de différentes vues d'une partie de corps du patient pendant les étapes du protocole dans un espace de travail (404) de l'interface utilisateur qui s'étend en travers de l'interface utilisateur, et dans lequel la pluralité d'images sont séparées d'autres éléments de l'interface utilisateur ;
afficher des informations relatives à une étape en cours du protocole illustrée par les images actuellement affichées dans l'espace de travail dans un espace d'informations (406) de l'interface utilisateur qui est positionnée en dessous de l'espace de travail et qui s'étend en travers de l'interface utilisateur ;
afficher une pluralité d'éléments de commande dans une barre de commande (408) de l'interface utilisateur qui est positionnée en dessous de l'espace d'informations et qui s'étend en travers de l'interface utilisateur ;
importer (174, 190) un profil de chirurgien du chirurgien, dans lequel le profil de chirurgien contient des données relatives à la manière avec laquelle le protocole chirurgical est personnalisé, de manière à permettre au système chirurgical assisté par ordinateur d'être personnalisé sur la base des préférences du chirurgien ; et
actionner (156, 158, 160, 162) le système chirurgical assisté par ordinateur en réponse à l'actionnement d'éléments de commande affichés dans l'interface utilisateur par le chirurgien conformément au protocole personnalisé.
